(19) Europäisches Patentamt
European Patent Office
Office européen des brevets

(11) EP 3 858 355 A1

(12) EUROPEAN PATENT APPLICATION
published in accordance with Art. 153(4) EPC

(43) Date of publication:
**04.08.2021 Bulletin 2021/31**

(21) Application number: **19864574.9**

(22) Date of filing: **24.09.2019**

(51) Int Cl.:
***A61K 31/4709*** *(2006.01)* ***A61P 9/10*** *(2006.01)*
***A61P 25/16*** *(2006.01)* ***A61P 25/28*** *(2006.01)*
***A61P 39/06*** *(2006.01)* ***A61P 43/00*** *(2006.01)*

(86) International application number:
**PCT/JP2019/037352**

(87) International publication number:
**WO 2020/067055 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.09.2018 JP 2018181074**

(71) Applicant: **Hayashibara Co., Ltd.**
**Okayama-shi, Okayama 702-8006 (JP)**

(72) Inventors:
- **OHTA Hitomi**
  **Okayama-shi Okayama 702-8006 (JP)**
- **MIYATA Satomi**
  **Okayama-shi Okayama 702-8006 (JP)**
- **MORIMOTO Takashi**
  **Okayama-shi Okayama 702-8006 (JP)**
- **HARASHIMA Akira**
  **Okayama-shi Okayama 702-8006 (JP)**
- **ARIYASU Toshio**
  **Okayama-shi Okayama 702-8006 (JP)**

(74) Representative: **Daniels, Jeffrey Nicholas**
**Page White & Farrer**
**Bedford House**
**John Street**
**London WC1N 2BF (GB)**

(54) **ANTI-NEURODEGENERATIVE DISEASE AGENT**

(57) The present invention addresses the problem of providing an orally-administrable anti-neurodegenerative disease agent having an action of accelerating neurocyte growth and an action of promoting neurite-outgrowth, and having excellent transfer properties to central nervous tissues. The problem is solved by providing an anti-neurodegenerative disease agent containing a compound represented by General formula 4 as an active ingredient, and having an action of accelerating neurocyte growth, an action of promoting neurite-outgrowth, and an action of scavenging reactive oxygen species. In General formula 4, $R_{10}$ and $R_{11}$ represent the same or different alkyl group having 2-8 carbon atoms, and the alkyl group may be either a straight chain or a branched chain. n represents an integer of 0 or 1, and $X_4^-$ represents a suitable counter anion that is pharmaceutically acceptable.

EP 3 858 355 A1

## Description

Technical Field

The present invention relates to an anti-neurodegenerative disease agent having an action of accelerating neurocyte growth of the central nervous system, an action of promoting neurite-outgrowth and an action of scavenging reactive oxygen species.

Background Art

Neurodegenerative diseases are pathological conditions caused by disruption of the neural network based on the systematic degeneration and loss of nerve cells, and many intractable diseases such as Alzheimer's disease, Parkinson's disease, Parkinson's syndrome, cerebrovascular dementia, frontotemporal lobe type dementia, amyotrophic lateral sclerosis, progressive supranuclear paralysis, Huntington's disease, and spinocerebellar degeneration are known.

It is expected that many molecular groups are complicatedly involved in the mechanism of neurodegenerative death, which is the cause of neurodegenerative diseases, and that their expression and functional abnormality are generated. Therefore, although intensive studies have been conducted on the molecular pathology, it is still only partially clarified, and a radical control method of the neurodegeneration has not been established.

In addition to the treatment of eliminating the etiology in neurodegenerative diseases, it is important to reconstitute the neural network. For example, in Alzheimer's disease, where cytotoxicity of amyloid β peptides is thought to be one of the causes, atrophy of neurites (axons and dendrites) and reduction of synapses are the initiating factors that impair neuronal function. Even after these events occur, it is said that neuronal function can be restored if partially degenerated neurons or surviving neurons are activated to extend neurites and restore synapses.

Neurodegenerative diseases represented by Alzheimer's disease and Parkinson's disease etc. are serious diseases that cause degeneration of nerve cells, and therapeutic agents containing various compounds as active ingredients have been proposed (see, Patent literatures 1 to 4) and neurite extenders have also been proposed (see, Patent literatures 5) in order to improve these diseases, pathologies associated therewith, and neurological dysfunction. The present applicant also found that cyanine dye compounds represented by following General formulae 1 to 3 have an excellent neurocyte growth accelerating action and a neurites-outgrowth promoting action based on screening of 239 kinds of compounds, and proposed as a new anti-neurodegenerative disease agent (Patent literature 6).

[Chem. 1]

General formula 1:

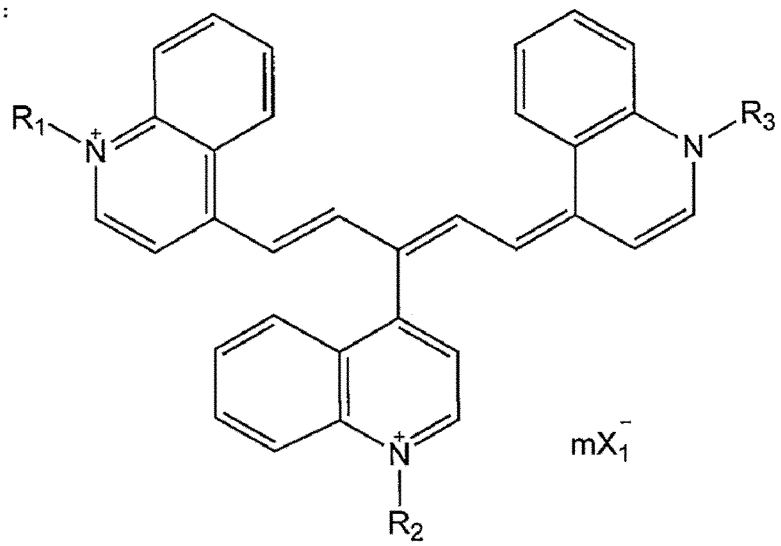

Wherein in General formula 1, $R_1$ to $R_3$ represent aliphatic hydrocarbon group that is the same as or different from each other. $X_1^-$ represents an appropriate counter anion, and m represents an integer of 1 or 2 that forms an electric charge for balancing with the electric charge of a cation part.

[Chem. 2]

General formula 2:

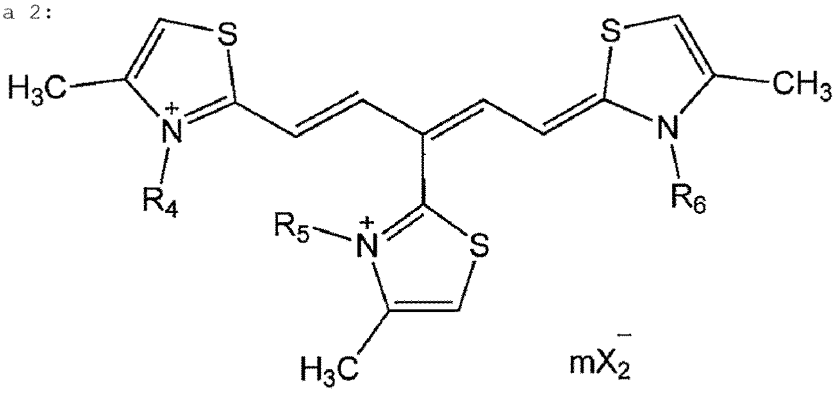

Wherein in General formula 2, $R_4$ to $R_6$ represent aliphatic hydrocarbon group that is the same as or different from each other. $X_2^-$ represents an appropriate counter anion, and m represents an integer of 1 or 2 that forms an electric charge for balancing with the electric charge of a cation part.

[Chem. 3]

General formula 3:

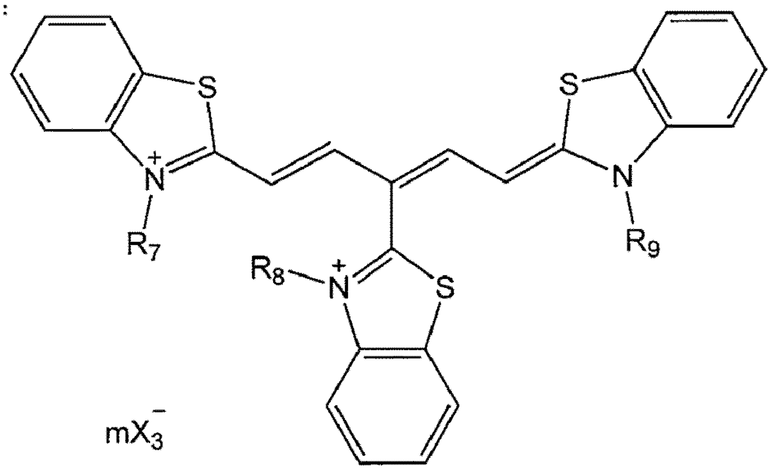

Wherein in General formula 3, $R_7$ to $R_9$ represent aliphatic hydrocarbon group that is the same as or different from each other. $X_3^-$ represents an appropriate counter anion, and m represents an integer of 1 or 2 that forms an electric charge for balancing with the electric charge of a cation part.

However, as a common point on the chemical structure of the dye compounds described above, they have large molecular weights and are bulky because their structures contain three heteroaromatic rings linked via a pentamethine chain. Therefore, there still remains problems in formulation in terms of translocation/penetration efficiency into the central nervous system, water solubility, and stability, and the possible dosage form as an agent is limited to parenteral administration.

In view of the fact that highly effective therapies have not yet been established for many neurodegenerative diseases, continuous research and development have being carried out in the medical and pharmaceutical industries. In the medical field, there is a demand for an orally administrable drug with a small physical and psychological burden on a patient, and even in the case of systemic administration such as subcutaneous or intravascular administration, there is a strong demand for the development of a new anti-neurodegenerative disease agent having a higher effect of acting on nerve cells of the central nervous system at a smaller dose or frequency.

Prior Art Literature

Patent Literature

Patent Literature 1: International Patent Publication No. WO97/030703 pamphlet
Patent Literature 2: Japanese Patent Kokai No. 1999-228417
Patent Literature 3: Japanese Patent Kokai No. 2006-143708
Patent Literature 4: Japanese Patent Kokai No. 2006-321737
Patent Literature 5: Japanese Patent Kokai No. 2002-234841
Patent Literature 6: International Patent Publication No. WO2010/087306 pamphlet

Disclosure of Invention

Object of the Invention

The object of the present invention is to provide an anti-neurodegenerative disease agent which has an action of accelerating neurocyte growth and an action of promoting neurite-outgrowth, and which is superior to the agent proposed by the present applicant in Patent literature 6 in transferability to central nervous system, and which can be orally administered.

As a result of extensive studies to solve the above object, the present inventors found that compounds represented by following General formula 4 have an excellent neurocyte growth accelerating action and a neurite-outgrowth action, and further an action of scavenging reactive oxygen species, and are excellent in transferability to central nervous system tissue.

[Chem. 4]

General formula 4:

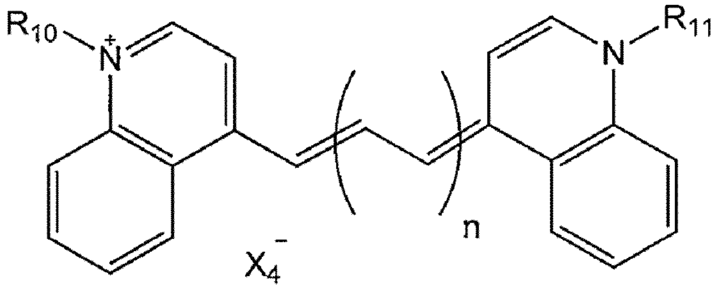

Wherein in General formula 4, $R_{10}$ and $R_{11}$ represent alkyl groups having 2 to 8 carbon atoms that are the same as or different from each other, and the alkyl groups may be linear or branched, n represents an integer of 0 or 1, and $X_4^-$ represents any pharmaceutically acceptable counter anion.

The above-mentioned compounds have a prominent feature that they have small molecular weights and excellent transferability to central nervous system tissue, while having a neurocyte growth accelerating effect and a neurite-outgrowth promoting effect substantially equal to or more than that of a cyanine dye compound (hereinafter referred to as "NK-4") represented by following Chemical formula 1 (also referred to as a compound represented by General formula 1), which is a typical active ingredient of an anti-neurodegenerative disease agent disclosed and proposed by the present applicant in Patent literature 6.

[Chem. 5]

Chemical formula 1:

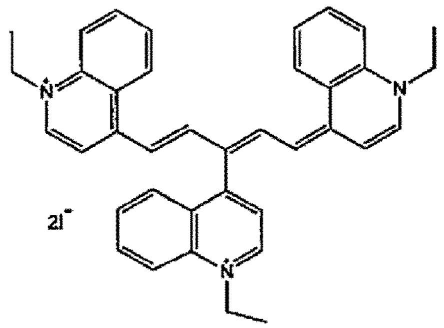

The compound NK-4 represented by above Chemical formula 1 is a carefully selected compound obtained by screening a library consisting of a large number of dye compounds using various evaluation systems, and has been considered that there is no other compound having an effect above or comparable to that of the neurocyte growth accelerating action and the neurite-outgrowth promoting action. Surprisingly, although the compound represented by General formula 4 is a smaller molecule than NK-4, it has a neurocyte growth accelerating action and neurite-outgrowth promoting action, which are the main action of NK-4, almost equal to or greater than NK-4, and in addition, it has an excellent heterogeneous properties, which were not found in the group of compounds disclosed in Patent literature 6, i.e., excellent transferability to central nervous tissue such as the brain, and further, it has a property of suppressing the production of active oxygen species within the cells when the neurocytes are exposed to oxidative stress. It is a finding that the compound represented by General formula 4 has such various excellent characteristics for the first time by the present inventors, and based on this finding, the present inventors have completed the present invention.

Means to Attain the Object

The present invention solves the above objects by providing an anti-neurodegenerative disease agent containing any of the following compounds represented by following General formula 4 as active ingredients and having an action of accelerating neurocyte growth and an action of promoting neurite-outgrowth of the central nervous system and an action of scavenging reactive oxygen species.

[Chem. 6]

General formula 4:

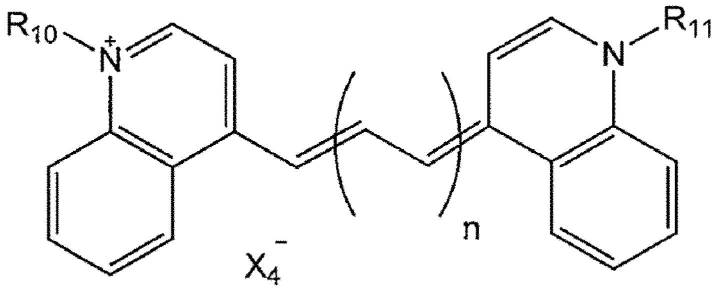

Wherein in General formula 4, $R_{10}$ and $R_{11}$ represent alkyl groups having 2 to 8 carbon atoms that are the same as or different from each other, and the alkyl groups may be linear or branched, n represents an integer of 0 or 1, and $X_4^-$ represents any pharmaceutically acceptable counter anion.

Effects of Invention

The anti-neurodegenerative disease agent of the present invention can be advantageously used for the treatment, alleviation, and prevention of neurodegenerative diseases by protecting nerve cells from oxidative stress and promoting neurocyte growth and neurite-outgrowth by administration orally or parenterally.

Mode for Carrying Out the Invention

1. Definitions of terms

In this specification, the following terms have the following meanings.

<Neurite(s)/Neurite-outgrowth promoting activity>

The term "neurite(s)" as referred to as in the present invention means axons and/or dendrites that extend from neurocyte. The term "neurite-outgrowth promoting activity" means an action of activating neurocytes to elongate axons and/or dendrites, as well as actions of inhibiting atrophy or reduction of neurites, accelerating synapse formation between neurocytes, and inhibiting synapse reduction.

<Neurodegeneration>

The term "neurodegeneration" as referred to as in the present invention means functional reduction, death or reduction (loss) of neurocytes in the central nerve system, and includes atrophy or reduction of neurites, reduction of synapses, functional reduction, death or decrease of glial cells, and death or degeneration of retinal cells.

2. Active Ingredients and Forms of the anti-neurodegenerative disease agent of the Present Invention

The anti-neurodegenerative disease agent of the present invention comprises a compound represented by following General formula 4 as an active ingredient.

[Chem. 7]

General formula 4:

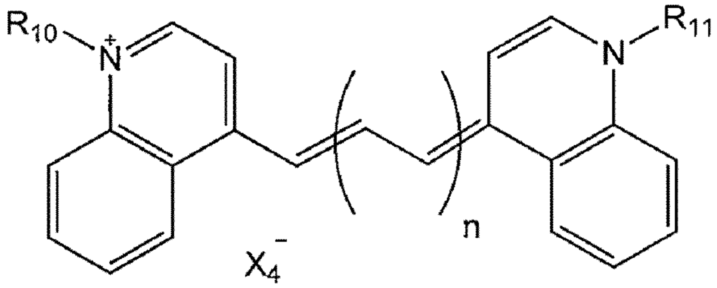

In General formula 4, $R_{10}$ and $R_{11}$ represent alkyl groups having 2 to 8 carbon atoms that are the same as or different from each other, and the alkyl groups may be linear or branched. n represents an integer of 0 or 1.

In General formula 4, $X_4^-$ represents a suitable pharmaceutically acceptable counter anion and typically selected from inorganic anions such as fluorine ion, chlorine ion, bromine ion, iodine ion, perchloric acid ion, periodic acid ion, hexafluorophosphoric ion, hexafluoroantimonate ion, hexafluorostannate ion, phosphoric acid ion, fluoroboric ion, and tetrafluoroborate ion; and from organic acid ions such as thiocyanic acid ion, benzene sulfonic acid ion, naphthalenesulfonic acid ion, naphthalenedisulfonic acid ion, p-toluenesulfonic acid ion, alkyl sulfonic acid ion, benzenecarboxylic acid ion, alkylcarboxylic acid ion, trihaloalkylcarboxylic acid ion, alkylsulfonate ion, trihaloalkyl sulfonate ion, nicotinic acid ion, and aspartic acid ion. In particular, chlorine ions, bromine ions, and iodine ions can be advantageously used.

Concrete examples of the compounds represented by General formula 4 which include compounds represented by Chemical formulae 2 to 11.

[Chem. 8]

Chemical formula 2:

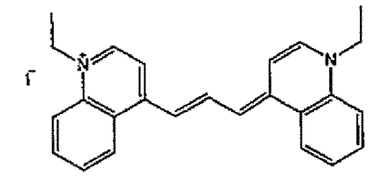

Chemical formula 3:

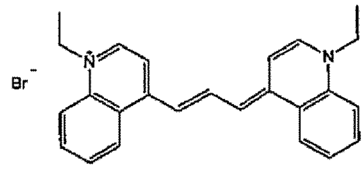

Chemical formula 4:

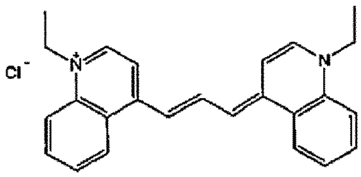

Chemical formula 5:

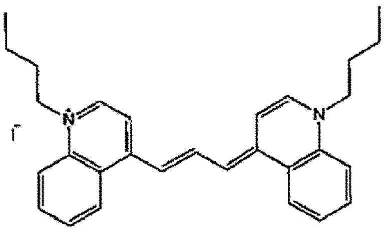

Chemical formula 6:

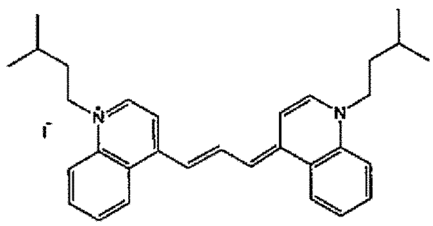

Chemical formula 7:

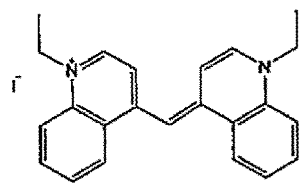

Chemical formula 8:

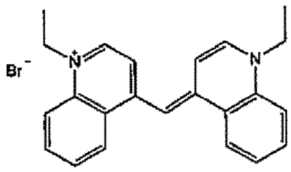

Chemical formula 9:

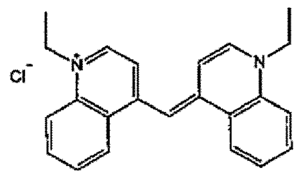

Chemical formula 10:

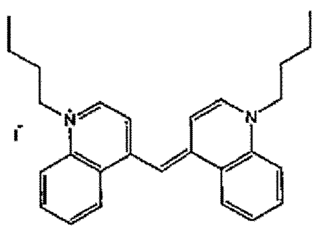

Chemical formula 11:

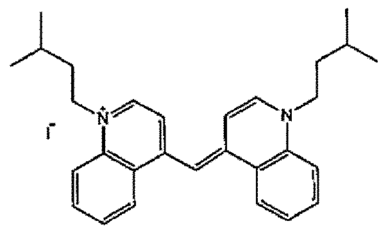

The compounds represented by above Chemical formulae 2 to 6 (hereinafter, may be called "NK-5", "NK-36", "NK-37", "NK-44" and "NK-45" in this order) are trimethine cyanine dyes corresponding to n = 1 in General formula 4, and the compounds represented by Chemical formulae 3 to 6 are analogs of the compound NK-5 represented by Chemical formula 2. For example, Chemical formula 3 (NK-36) and Chemical formula 4 (NK-37) are compounds in which the iodide ion, which is the counter anion of NK-5, is replaced with bromine ion and chlorine ion, respectively, and Chemical formula 5 (NK-44) and the compound represented by Chemical formula 6 (NK-45) are compounds in which the N-alkyl chain of NK-5 is replaced with an n-butyl group and an isopentyl group, respectively, from an ethyl group.

Similarly, the compounds represented by Chemical formulae 7 to 11 (hereinafter may be called "NK-6", "NK-1496", "NK-1495", "NK-10745" and "NK-1486" in this order) are monomethine cyanine dyes corresponding to n = 0 in General formula 4, and Chemical formulae 8 to 11 are analogs of the compound NK-6 represented by Chemical formula 7. Similar to the above, the compounds represented by Chemical formulae 8 and 9 are compounds in which the iodine

ion, which is the counter anion of NK-6, is replaced with the bromine ion and the chlorine ion, respectively, and Chemical formula 10 (NK-10745) and Chemical formula 11 (NK-1486) are compounds in which the N-alkyl chain of NK-6 is replaced by a n-butyl group and an isopentyl group from an ethyl group, respectively.

The structure of the compounds corresponding to the compound number starting with NK (hereinafter referred to as "NK number") in this specification are also described in, for example, "Kankoso-Hyo" (Table of Photosensitive Dyes), published by Kankoshikiso Kenkyu-Sho, Okayama, Japan, (1969), and "CHEMICAL ABSTRACT Index Guide (N-Z), pp. 1531G-1536G, 1994. The Chemical formula number in this specification is described together with the NK number in the experimental results described later.

In addition, methods for synthesizing these compounds are also described, for example, in "Photosensitive dyes -their mysterious actions and various functions-", published by Sangyo Tosho Co., Ltd., pp. 24 to 30 (1997), and "The Chemistry of Synthetic Dyes", published by Academic Press, Vol. 2, p. 1143 (1952) and Vol. 4, p. 212 (1971).

The compound represented by General formula 4 has a growth acceleration activity and a neurite-outgrowth promoting activity on nerve cells, and has a function of protecting nerve cells from reactive oxygen species (hereinafter, referred to simply as "ROS") induced in cells by oxidative stimulation to suppress cell death and atrophy of neurites, and therefore is desirable as an active ingredient of the anti-neurodegenerative disease agent of the present invention. The chain length of the N-alkyl group represented by $R_{10}$ and $R_{11}$ is usually in the range of 2 to 8 carbon atoms, preferably 2 to 5 carbon atoms, and more preferably 2 to 4 carbon atoms.

NK-5 (Chemical formula 2) and its analogs (Chemical formulae 3 to 6) are more desirable from the viewpoint of the intensity of the neurocyte growth acceleration activity, the neurite-outgrowth action, and the ROS-scavenging action. In view of water solubility, brain permeability, ease of formulation, and the like, it is particularly desirable to use NK-5 (Chemical formula 2) and its analogs (Chemical formulae 3 to 6) and NK-6 (Chemical formula 7) and its analogs (Chemical formulae 8 to 11). In addition, NK-6 (Chemical formula 7) and its analogs (Chemical formulae 8 to 11) may be particularly advantageously used when stability and persistence of the efficacy is an important point.

As described above, in the compound group represented by General formula 4, each compound has a plurality of physiological and/or chemical actions, and the action intensity and chemical properties thereof are individually different, it is also optional to exhibit a more preferable effect according to the target disease by combining two or more kinds of compounds according to the action spectrum and physical properties of the compounds. For example, when designing an orally-administered preparation, by considering both the action and stability thereof, it can be possible to select a compound with strong action intensity and another compound with high stability, and mix them appropriately in an arbitrary concentration range, to make a more effective agent. For example, when a trimethine dye represented by NK-5 (Chemical formula 2) corresponding to the case of n=1 in General formula 4 and a monomethine dye represented by NK-6 (Chemical formula 7) corresponding to the case of n=0 in General formula 4 are used as a mixed preparation, there is no limitation on the mixing ratio thereof as long as the desired effect can be obtained. The molar ratio of the trimethine dye and the monomethine dye is usually suitable in the range of 1:10 to 10:1

The compound represented by General formula 4 used as the active ingredient of the anti-neurodegenerative disease agent of the present invention should not be restricted to specific origins and preparation methods.

The anti-neurodegenerative disease agent of the present invention contains, as an active ingredient, one or more compounds represented by General formula 4, preferably a trimethine cyanine dye and/or a monomethine cyanine dye represented by any of Chemical formulae 2 to 11.

The anti-neurodegenerative agents of the present invention are provided in the form of oral or parenteral preparations and, if necessary, one or more ingredients which are acceptable for pharmaceutical engineering and in the fields of food products, cosmetics, pharmaceuticals, or quasi-drugs are optionally incorporated into the anti-neurodegenerative agents, in addition to the compound represented by General formula 4, which is the active ingredient.

Examples of the ingredients acceptable for pharmaceutical engineering include additives, excipients, disintegrants, lubricants, binders, stabilizers, surfactants, preservatives (antimicrobials), flavors, thickeners, antioxidants, chelating agents, vitamins, amino acids, aqueous media, saccharides, water-soluble polymers, pH adjusters, foaming agents, additives for pharmaceuticals/quasi-drugs/cosmetics/food products, effective ingredients for pharmaceuticals/quasi-drugs, etc., one or more of which can be appropriately incorporated in combination to prepare the agent of the present invention depending on its intended dosage form.

The anti-neurodegenerative disease agent of the present invention can also be advantageously used in combination with a neurite extension agent containing a substance other than the compound represented by General formula 4 as an active ingredient, or in combination with a therapeutic agent for a neurodegenerative disease or neurofunctional disorders. These medications can be administered separately or in a mixed formulation with the effective ingredient(s) of the present invention.

There is no limitation on the route of administration of the anti-neurodegenerative disease agent of the present invention, and it is preferably provided in the form of an oral preparation in consideration of the physical and mental burden of the patient. It is also optional to be provided in the form of a preparation for injection for parenteral use, or the like, when a higher efficacy is required. The compound represented by General formula 4 which is an active ingredient

can be incorporated in any step from the stage of the raw material to the completion of the final product in consideration of the composition of the dosage preparation to be administered as a subject and the purpose of use thereof. Examples of the methods thereof can be appropriately selected from one or more methods such as mixing, stirring, kneading, granulation, dissolving, melting, dispersing, suspending, emulsifying, reverse micellization, hot melt, permeation, crystallizing, spreading, applying, spraying, coating, injecting, soaking, solidifying, supporting, etc.

As described above, the anti-neurodegenerative disease agent of the present invention can be provided in the form of a composition for oral intake. In such cases, there is no particular restriction on the dosage form, and it can be taken in the form of solid, powder, granule, tablet, liquid, syrup, paste, emulsion, capsule, film, etc. and it is also possible to take it by mixing it with ordinary food and beverages.

In the case of parenteral medicaments such as injection medicaments, they can be in the form of a dried or liquid medicament because, depending on diseases or symptoms to be applied, they are usually dissolved in pyrogen-free aqueous medium before being injected intradermally, subcutaneously, intramuscularly, intracorporeally (intrapleurally, intraperitoneally, etc.), intravascularly, or intracerebrally including intraspinally. In the case of dried medicaments, they can be used after being dissolved in aqueous solvents such as purified water for injection, physiological saline, and glucose solution. In the case of liquid medicaments, they can be administered intact or used after being added to parenteral fluid, perfusion solution, peritoneal dialysate, etc. If solubility in solvents or aqueous media is a problem, or if a sustained-release formulation is to be prepared, amphiphilic solvents, oily base materials, or emulsifiers can be used to increase the solubility of the active ingredient in the solvent. The effective ingredients can be administered after being encapsulated into liposome, etc.

In addition to oral and injectable formulations, the anti-neurodegenerative agents can also be used in the form of poultice (happ agent), transpulmonary inhalation sprays, and sustained release formulations that are implanted in the body, such as subcutaneously. They can also be used to treat pets and other non-human animals that have developed neurodegenerative diseases, and as prophylactic or therapeutic agents for conditions and neurological dysfunction associated with neurodegenerative diseases.

The anti-neurodegenerative disease agent of the present invention thus produced is a safe medicament without causing serious side effects even if it is used continuously for a long period of time.

Depending on pathological conditions or symptoms, the anti-neurodegenerative disease agent of the present invention can be administered with a prescribed amount of dose every day or at an interval of one or more days and at a dose frequency of once or several times a day. The daily intake or dose should not specifically be restricted as long as it attains a desired function and effect; usually, in the case of oral administration, the compounds represented by General formula 4 can be administered in total at a dose of 0.02 mg/kg body weight/day or more, preferably, 0.2 to 50 mg/kg body weight/day, and most preferably, 1 to 10 mg/kg body weight/day. In the case of intravenous administration including instillation, as well as subcutaneous and intraperitoneal administrations, the compounds represented by General formula 4 can be administered in total at a dose of 0.002 mg/kg body weight/day or more, preferably, 0.02 to 5 mg/kg body weight/day, and most preferably, 0.1 to 1 mg/kg body weight/day. If the daily intake or dosage is less than the lower limit of the specified amount, the intended effect may not be exhibited, and even if the upper limit is exceeded, the effect commensurate with the intake may not be exhibited. When administered with an expectation of acting as a radical scavenger, the anti-neurodegenerative disease agent of the present invention can be arbitrarily administered at a higher dose than the above-identified doses. In addition, the administration period of the anti-neurodegenerative disease agent of the present invention can be controlled depending on the objective diseases, pathological conditions, and symptoms of the subject, and in the case of an acute disease, the administration may be performed until the symptom improves or disappears, and in the case of a chronic disease such as dementia, it is desirable to continue the administration even if the symptom improves or disappears.

The anti-neurodegenerative disease agent of the present invention treats, eases or prevents neurodegenerative diseases, particularly diseases induced by central nervous denaturation because it can protect the brain and/or neurocytes from injury factors to inhibit denaturation of neurocytes, activate neurocytes, promote neurite-outgrowth or inhibit neurite atrophy, and prolong neurocyte survival or inhibit neurocyte denaturation. The term "neurodegenerative diseases" as referred to as in the present invention includes all diseases which accompany denaturation of neurocytes in central nerves (e.g., cranial nerve and spinal nerve), and/or peripheral nerves (e.g., autonomic nerve such as sympathetic nerve and parasympathetic nerve, motor nerve system, and sensory nerve system), and it should not be restricted by its causatives.

Preferred examples of neurodegenerative diseases, to which the agent of anti-neurodegenerative disease of the present invention is applicable, include Alzheimer's disease, Parkinson's disease, Parkinson's syndrome, Huntington's disease, senile dementia, spinocerebellar degeneration, amyotrophic lateral sclerosis, demyelinating disease (e.g., multiple sclerosis, etc.), cerebrovascular disease (e.g., cerebrovascular stroke, cerebral infarction (e.g., cerebral thrombosis, cerebral embolism, etc.)), transient cerebral ischemic attack, neurologic dysfunction accompanied by cerebral hemorrhage (e.g., hypertensive intracerebral hemorrhage, subarachnoid cerebral hemorrhage, etc.), brain tumor, traumatic shock, head injury and/or traumatic cerebrospinal injury (e.g., brain contusion etc.), encephalomyelopathy accompanied by infectious diseases (e.g., meningitis, influenza encephalitis/encephalopathy, Creutzfeldt-Jakob diseases, agnosia induced by AIDS encephalopathy, etc.), epilepsy, those diseases originated from neurodegeneration in the central nerve system; more preferably, for example, Alzheimer's disease, Parkinson's disease, Parkinson's syndrome, amyotrophic lateral sclerosis associated neurologic dysfunction, ischemic brain disease such as cerebrovascular accident and transient ischemic attack.

In addition, the anti-neurodegenerative disease agent of the present invention also can treat neurologic dysfunction by activating neurocytes, elongating neurites, promoting synapse formation, etc. Therefore, the anti-neurodegenerative disease agent of the present invention can be advantageously used as a neurocyte protective agent, neurocyte activator, neurite-outgrowth promoting agent, neurite atrophy inhibitor, inhibitor for Purkinje's cell degeneration/dropout, therapeutic agent for pathema accompanied by neurodegenerative diseases (for example tremor, rigidity, akinesia, paralysis, bradykinesia, posture reflex disorder, autonomic neuropathy, rush phenomenon, gait disorder, depression, memory disorder, muscle atrophy, muscle weakness, upper/lower limb dysfunction, articulation disorder, dysphagia, respiratory disorder, numbness and paralysis, etc.) or for neurologic dysfunction. The term "treatment of neurodegenerative diseases and neurologic dysfunction" as referred to as in the present invention means progression inhibition to prevent progression of pathema accompanied by neurodegeneration, and prevention of the onset of diseases, as well as so called therapy to allow pathema or functional dysfunction inherent to neurodegeneration to direct to curing.

Further, the anti-neurodegenerative disease agent of the present invention can effectively reduce free radicals, which generated in vivo, including hydroxy radicals. In addition, since the anti-neurodegenerative disease agent of the present invention can scavenge ROS produced by metabolic activities by being transferred into cells and/or tissues, the accumulation of oxidative damage in the intracellular organ can be minimized, and the function of the cell body can be well maintained. Thus, it can be advantageously used as a prophylactic or therapeutic agent for diseases and pathema inherent to free radicals or lipoperoxide generated by recirculation after ischemia in vessels and organs other than brain, as well as tissues; inflammatory diseases including immunopathy, allergy, and tumors; infectious diseases; drugs; radiations; and physical stimulations. More specifically, in addition to a prophylactic and therapeutic agent for the above-identified neurodegenerative diseases, the agent can be advantageously used as, for example, a brain protective agent, inhibitor of a brain oxidative injury (neurocyte, vascular endothelial cells), ischemic brain injury inhibitor, cerebral infarction development inhibitor, cerebral edema inhibitor, a late-onset neuronal death inhibitor, a brain function normalizing agent, oxidative stress inhibitor, anti-ulcer agent, hyperglycemia inhibitor, and prophylactic and therapeutic agents for ocular diseases such as cataract and corneal injury; organ transplant preservative; necrosis inhibitor for transplanted tissues (including the skin) /organs; prophylactic and therapeutic agents for organ disorders such as nephropathy induced by acute renal failure/chemicals, skin tissue damage, lung injury, liver fibrosis, functional disorder of skin tissue induced by chemical substances, endotoxin and burns etc., liver damage induced by ischemia, spinal cord injury, vessel wall disability such as artery, muscle disability such as myocardia, prophylactic and therapeutic agents for disorders of various organs such as renal tubulointerstitial disorders; cytotoxic marker inhibitors, myocarditis, pancreatitis, prophylactic and therapeutic agents for radiation injury; anti-tumor agents, tumor metastasis inhibitors, cytotoxic marker inhibitors, myocarditis, pancreatitis, enteritis; prophylactic and therapeutic agents for inflammatory diseases of various tissues and organs such as joints and allergies and associated tissue disorders; inhibitors for sensory cell, sensory nerve, and sensory organ disorders such as optic cell disorders, optic nerve disorders, retinal disorders, auditory cell disorders, auditory nerve disorders, etc.; prophylactic and therapeutic agents for medicinal poisoning induced by pesticides and organic solvents; Ca/Na exchange inhibitor, prophylactic and therapeutic agents for pain and itching; protein kinase stimulant; prophylactic and therapeutic agents for mitochondrial encephalomyopathy; prophylactic and therapeutic agents for arterial occlusion/stenosis; blood-brain barrier disruption inhibitor; therapeutic agent for drug addictions; apoptosis inhibitor; inhibitor for lipid peroxide production in organs and tissues; and radical scavengers such as superoxide, hydroxyl radicals and peroxyl radicals.

The following experiments explain the present invention in more detail. In each table showing the experimental results, the correspondence between the chemical formula number and the NK number in the present specification is also shown.

Experiment 1: Effect on neurocytes

Neuronal cells are known to be very vulnerable to injury induced by nutrient starvation and reactive oxygen. Since substances that promote the proliferation and differentiation of neuronal cells can be expected to have the effect of suppressing neuronal cell death caused by neurodegenerative diseases, and can be candidates for therapeutic agents for diseases, a screening test for compounds having neurocyte growth acceleration activity and neurite-outgrowth promoting activity was carried out by using PC12 cells, which are model cells of the nervous system, in accordance with the method disclosed in Patent literature 6 as follows.

Experiment 1-1: Screening of dye compounds having neurocyte growth acceleration activity and neurite-outgrowth promoting activity 1

As disclosed by the present inventors in Patent literature 6, some cyanine dyes such as NK-4 represented by Chemical formula 1 have a neurocyte growth acceleration activity and a neurite-outgrowth promoting activity. With the aim of developing an effective drug having a higher bioavailability, a screening was carried out using NK-4 as a positive control. As a model of the nerve cell, the NGF (nerve growth factor) highly sensitive strain of PC12 cell derived from the rat adrenal pheochromocytoma (hereinafter referred to as "PC12-HS cell"; JCRB cell bank, National Research and Development Institute of Health and Nutrition), which has been widely used as a suitable model for research on human neurodegenerative disease, was used. As the test compound, 10 kinds of trimethine cyanine dye compounds; NK-5 (Chemical formula 2), NK-36 (Chemical formula 3), NK-44 (Chemical formula 5) and dye compounds represented by Chemical formulae 12 to 18, and NK-6 (Chemical formula 7), which is a monomethine cyanine dye, were selected. In addition, two kinds of ethylene cross-linked dyes (Chemical formulae 19 and 20), 15 kinds of monomethine cyanine dyes (Chemical formulae 21 to 35), and seven kinds of trimethine cyanine dyes (Chemical formulae 36 to 42), which were expected to have a good neurocyte growth acceleration activity and/or neurite-outgrowth promoting activity in the screening disclosed in Patent literature 6, were also re-evaluated for comparison. The Chemical formulae 12 to 42 are shown below.

[Chem. 9]

Chemical formula 12:

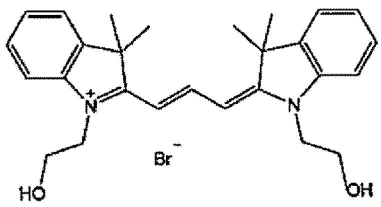

Chemical formula 13:

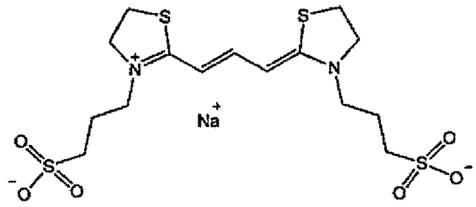

Chemical formula 14:

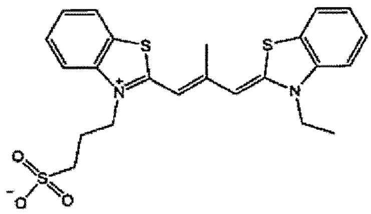

Chemical formula 15:

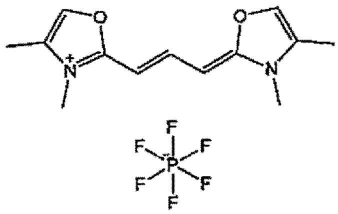

Chemical formula 16:

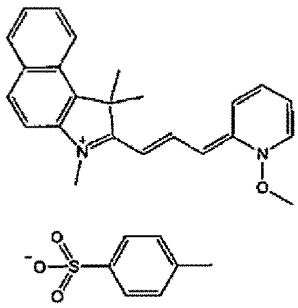

Chemical formula 17:

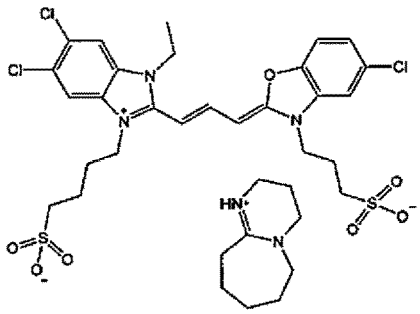

Chemical formula 18:

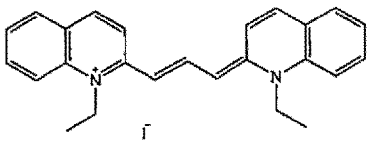

[Chem. 10]

Chemical formula 19:

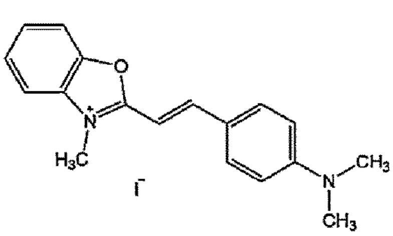

Chemical formula 20:

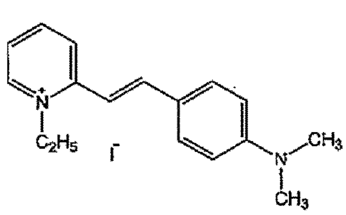

[Chem. 11]

Chemical formula 21:

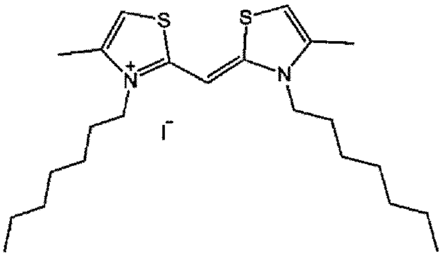

Chemical formula 22:

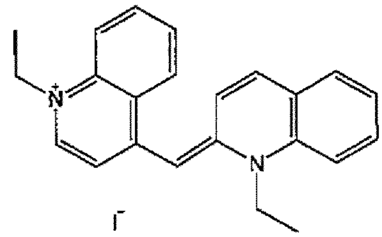

Chemical formula 23:

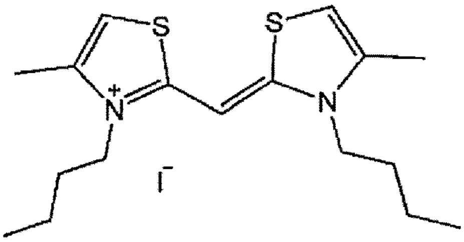

Chemical formula 24:

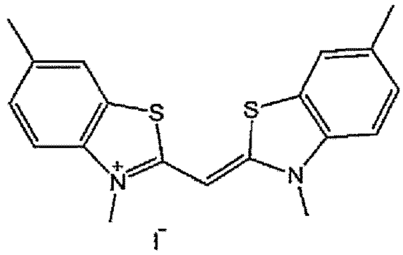

Chemical formula 25:

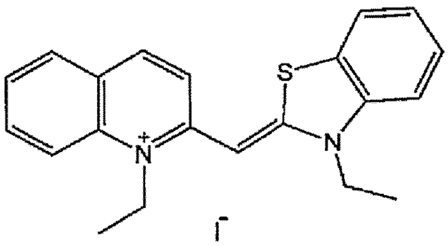

Chemical formula 26:

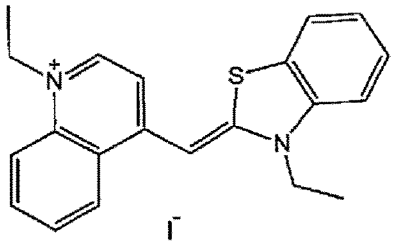

Chemical formula 27:

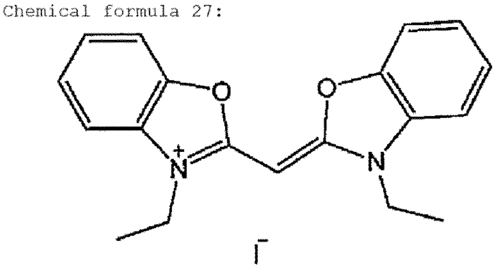

Chemical formula 28:

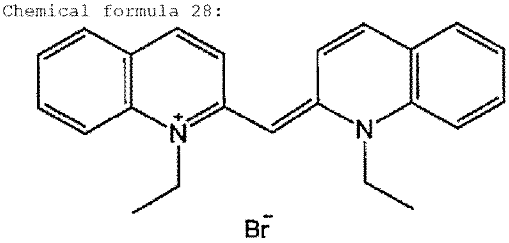

Chemical formula 29:

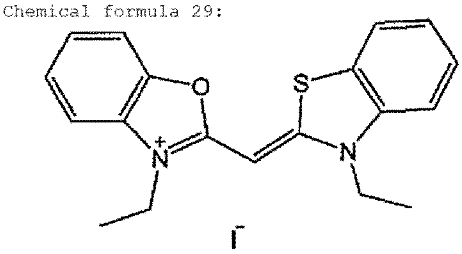

Chemical formula 30:

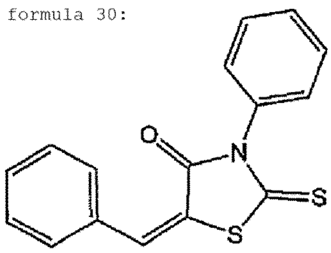

[Chem. 12]

Chemical formula 31:

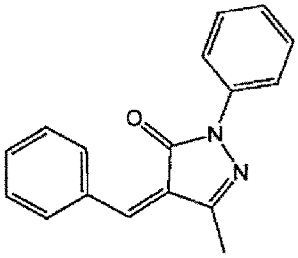

Chemical formula 32:

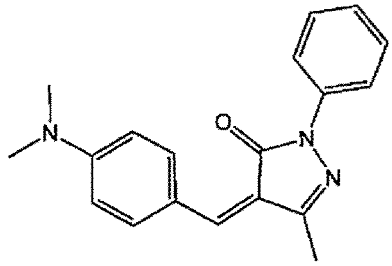

Chemical formula 33:

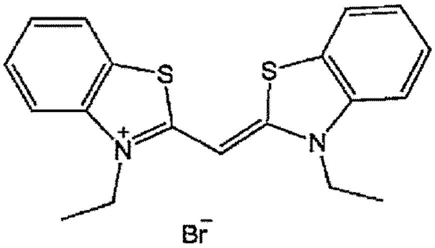

Chemical formula 34:

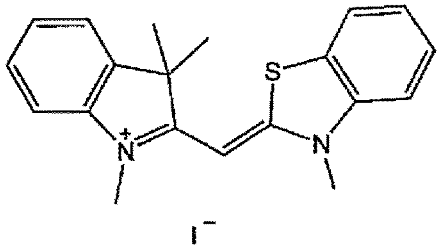

Chemical formula 35:

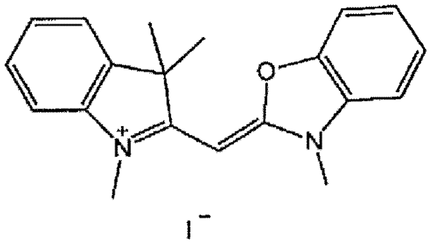

[Chem. 13]

Chemical formula 36:

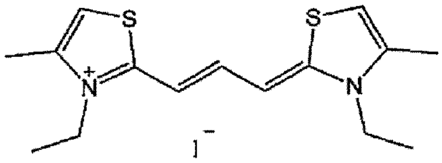

Chemical formula 37:

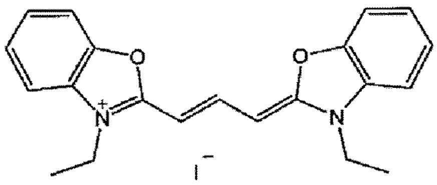

Chemical formula 38:

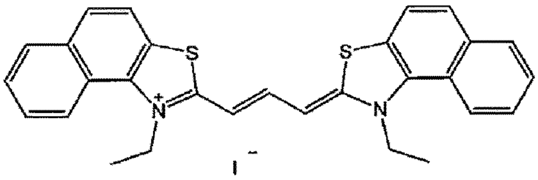

Chemical formula 39:

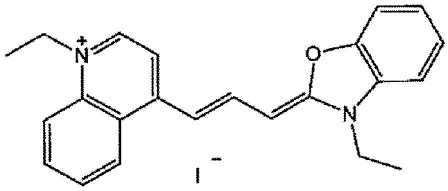

Chemical formula 40:

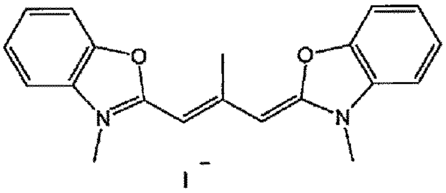

Chemical formula 41:

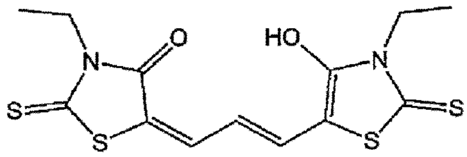

Chemical formula 42:

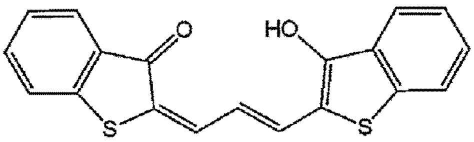

<Test samples>

The cyanine dye compounds subjected to the test (purity 97% by mass or more, synthesized by Hayashibara Co., Ltd., Okayama, Japan) were individually dissolved in DMSO (product number "D8418", commercialized by SIGMA Corporation) at a concentration of 5 mg/ml, and the solutions were then filtered through DMSO-resistant membrane ("Millex-LGS LLG025SS", commercialized by Millipore Corporation) and stored at 25 °C in a dark condition. At the time of use, they were further diluted with Dulbecco's Medium (hereinafter, abbreviated as "D-MEM medium", commercialized by Nissui Pharmaceutical Co., Ltd.) supplemented by 10% by volume of fetal bovine serum (FBS), and subjected to the test. It was confirmed beforehand that DMSO contained in the test samples did not affect the testing system, when the dye compound dissolved in DMSO was further diluted in D-MEM medium to the concentrations for use in the test.

Hereinafter, the neurocyte growth acceleration activity and neurite-outgrowth promoting activity of cyanine dye compounds on PC12-HS cells in the presence of NGF were evaluated, respectively.

<Evaluation method A: Evaluation method of neurocyte growth acceleration activity>

PC12-HS cells were subjected to tests after frozen stock cultured cells were thawed and cultured with a D-MEM medium supplemented with 10% by volume of FBS (Hereinafter, when the term "culture" is simply used, it means that the cells are statically cultured in an incubator controlled at 37 °C under 5% by volume of $CO_2$ conditions). Cells used in the test were collected using 0.25 % by weight of trypsin solution, then diluted with a D-MEM medium supplemented with 10% by volume of FBS (hereinafter referred to as "culture medium"), seeded into collagen-coated 96-well plates (commercialized by Falcon, Tokyo, Japan, "Microtest Plate, Cell Culture, Flat Bottom") to obtain $5 \times 10^3$ cells/100 μl/well, and cultured for 24 hours (hereinafter referred to as "pre-culture"). Then, the above test samples were diluted with a culture medium, and 100 μl of diluted solution of the test compounds were added to each well so that the final concentration

of the test compounds in the wells became 50 ng/ml, and the cells were cultured for another three days. On day 3, the cultures supernatant was removed from each well, 200 μl of culture medium containing 10% by volume of Alamar blue reagents (commercialized by Trek Diagnostic Systems Inc., Ohio, USA) were added to each well, and the cells were cultured for 6 hours. After culturing for 6 hours, the cells were measured for fluorescent intensity at a wavelength of 544 - 590 nm by "FlexStation 3", a product name of a fluorescent plate reader (commercialized by Japan Molecular Devices Corporation, Tokyo, Japan). The action of cell growth promotion in each test compound was expressed as the cell proliferation rate (%), and when the value of the fluorescence intensity of the negative control in which 100 μl/well of the culture medium containing no test compound was added, was set to 100%, the relative value of 130% to 169% was judged to be equivalent to NK-4 ("circle"), and 170% or higher was judged to be significantly stronger than NK-4 ("double circle"), and is shown in Table 1. Measurements were performed for each test compound in triplicate, and the mean values were shown. Table 1 also shows the evaluation results of the neurite-outgrowth promoting activity by the evaluation method B described later.

<Evaluation method B: Evaluation method of neurite-outgrowth promoting activity >

As in the case of evaluation of neurocyte growth accelerating activity (Evaluation method A), 50 μl/well of each test compound solution, prepared by diluting with the culture medium to give a final concentration of 500 ng/ml, and the culture medium containing 20 ng/ml of mouse NGF (mouse derived, final concentration 10ng/ml, commercialized by Chemicon International, Inc., CA, USA) (50 μl/well) were added to the precultured PC12-HS cells in each well, and then cultured for three days. Culture medium was removed on day 3 of culture, and cells were fixed by adding 100 μl/well of 10% by volume of glutaraldehyde and standing for 20 minutes at room temperature. As a negative control, PC12-HS cells, cultured for three days with culture medium, were fixed with glutaraldehyde similarly as above. Fixed cells were observed under a microscope to assess the neurite-outgrowth. The neurite-outgrowth rate (%) was determined by observing the cells using a microscope by adjusting the magnitude to give about 100 cells in one visual field, counting the number of cells having neurites twice or more in length of the cell body (major axis), dividing by the total number of cells in the same visual field, and multiplying by 100. Measurements were carried out at three points for each test compound. When the mean neurite-outgrowth rate was 25 to 44%, it was judged to be equivalent to the positive control NK-4 ("circle"), and 45% or more was judged to be more potent than NK-4 ("double circle"), and the results are shown in Table 1. In this experiment, the neurite-outgrowth rate was 5% or less when NGF was added (final concentration 10ng/ml) without the addition of the test compound.

Table 1

| Structural classification | | Compound (NK No.) | Chemical formula No. | Mw (Da) | | Action on PC12-HS | |
|---|---|---|---|---|---|---|---|
| # of Rings | Linker | | | Whole | Cation | growth acceleration (50 ng/ml) | neurite-outgrowth promotion (500 ng/ml) |
| 3 | pentamethine | 4 | 1 | 780.52 | 662.61 | ○ | ○ |

(continued)

| Structural classification | | Compound (NK No.) | Chemical formula No. | Mw (Da) | | Action on PC12-HS | |
|---|---|---|---|---|---|---|---|
| # of Rings | Linker | | | Whole | Cation | growth acceleration (50 ng/ml) | neurite-outgrowth promotion (500 ng/ml) |
| 2 | ethylene | 528 | 19 | 406.27 | 279.36 | ○ | ○ |
| | | 557 | 20 | 380.27 | 253.36 | | ○ |
| | monomethine | 6 | 7 | 454.35 | 327.44 | ○ | ◎ |
| | | 266 | 21 | 534.6 | 407.69 | | ○ |
| | | 276 | 22 | 454.35 | 327.44 | | ○ |
| | | 399 | 23 | 450.44 | 323.53 | | ○ |
| | | 594 | 24 | 466.4 | 339.49 | | ○ |
| | | 723 | 25 | 460.37 | 333.46 | | ○ |
| | | 750 | 26 | 460.37 | 460.37 | | ○ |
| | | 863 | 27 | 434.28 | 307.37 | | ○ |
| | | 1046 | 28 | 407.35 | 327.44 | | ○ |
| | | 1049 | 29 | 450,33 | 323.42 | | ○ |
| | | 1075 | 30 | 297.39 | 297.39 | ○ | |
| | | 1076 | 31 | 262.3 | 262.3 | ○ | |
| | | 1107 | 32 | 305.37 | 305.37 | ○ | |
| | | - 1560 | 33 | 419.4 | 339.49 | | ○ |
| | | 1570 | 34 | 448.36 | 321.45 | | ○ |
| | | 1684 | 35 | 432.29 | 305.38 | | ○ |
| | trimethine | 3 | 18 | 480.38 | 353.48 | | |
| | | 5 | 2 | 480.38 | 353.48 | ○ | ◎ |
| | | 36 | 3 | 433.38 | 353.48 | ○ | ○ |
| | | 44 | 5 | 536.49 | 409.59 | ◎ | ○ |
| | | 67 | 36 | 420.37 | 293.46 | | ○ |
| | | 85 | 37 | 460.3 | 333.39 | | ○ |
| | | 382 | 38 | 592.55 | 465.64 | | ○ |
| | | 741 | 39 | 470.35 | 343.44 | | ○ |
| | | 1300 | 40 | 446.28 | 319.37 | | ○ |
| | | 1451 | 41 | 358.52 | 358.52 | ○ | |
| | | 1741 | 42 | 336.42 | 336.42 | ○ | |
| | | 1883 | 12 | 497.46 | 417.56 | | |
| | | 3134 | 13 | 478.6 | 457.63 | ○ | |
| | | 4781 | 14 | 472.64 | 472.64 | | ◎ |
| | | 10089 | 15 | 378.25 | 233.29 | ○ | |
| | | 10214 | 16 | 528.66 | 357.47 | | ◎ |
| | | 10358 | 17 | 817.24 | 666.01 | ○ | |

Since the screening results shown in Table 1 do not consider the optimum concentration of each compound, it is not possible to make a strict comparison of the intensity of the action. However, NK-528 (Chemical formula 19) and NK-36 (Chemical formula 3) were evaluated to be equivalent ("circle") in both neurocyte growth acceleration activity and neurite-outgrowth promoting activity as compared with NK-4, which is most preferable as an active ingredient of an anti-neurodegenerative disease agent in Patent literature 6. In addition, NK-5 (Chemical formula 2) and NK-6 (Chemical formula 7) showed a neurocyte growth acceleration activity equivalent to NK-4 ("circle"), and also showed a stronger neurite-outgrowth promoting activity than NK-4 ("double circle"). On the contrary, NK-44 (Chemical formula 5) was found as a compound evaluated to show a neurocyte growth acceleration activity equivalent to NK-4 ("circle") and to show a neurite-outgrowth promoting activity stronger than that of NK-4 ("double circle"). The other compounds did not reach NK-4 potency in either or both evaluations, whereas NK-4781 (Chemical formula 14) and NK-10214 (Chemical formula 16) were evaluated to have a neurite-outgrowth promoting activity superior to NK-4 ("double circle"). This indicates that the ethylene-linked NK-528 (Chemical formula 19), the monomethine-linked NK-6 (Chemical formula 7) and the trimethine-linked NK-5 (Chemical formula 2), NK-36 (Chemical formula 3) and NK-44 (Chemical formula 5) have an activity equivalent to or higher than NK-4 in both the neurocyte growth acceleration activity and the neurite-outgrowth promoting activity, and these compounds may be useful as active ingredients for anti-neurodegenerative diseases. Incidentally, all the compounds which have been shown to be useful as active ingredients of these anti-neurodegenerative disease agents were compounds represented by General formula 4, except for NK-528 (Chemical formula 19). In addition, the trimethine compounds represented by Chemical formulae 13 to 17 were also evaluated to be equivalent to NK-4 ("circle") or higher ("double circle") in either the neurocyte growth acceleration activity and the neurite-outgrowth promoting activity, so these compounds are considered to be useful as active ingredients of an anti-neurodegenerative disease agents.

Experiment 1-2: Screening of dye compounds having neurocyte growth acceleration activity and neurite-outgrowth promoting activity 2

Based on the above-mentioned screening results at a single concentration (Experiment 1-1), eleven dye compounds including NK-4 were selected and the relationship between their molar concentration and the action intensity was confirmed. The method was based on Experiment 1-1, except the concentrations of the test compounds were set to three doses of 40 nM, 200 nM and 1,000 nM for evaluation method A (neurocyte growth accelerating effect), and two doses of 200 nM and 1,000 nM for evaluation method B (neurite outgrowth promoting effect). Measurements were performed in triplicate for each concentration of each test compound in both evaluation methods A and B. The result of the evaluation method A was expressed as a cell proliferation rate (%) by determined a relative percentage (%) when the value of the negative control to which the test compound was not added, was regarded as 100%. In evaluation method B, the number of cells having neurites twice or more in length of the cell body (major axis) in the total cell in the visual field (containing about 100 cells) was expressed as a relative percentage and used as the neurite-outgrowth promoting rate (%). In this experiment, the neurite-outgrowth rate was 5% or less when only NGF was added (final concentration 10 ng/ml) without the addition of the test compound.

Table 2

| Compound (NK No.) | Chemical formula No. | Cell proliferation rate (%) | | | Neurite-outgrowth rate (%) | |
|---|---|---|---|---|---|---|
| | | 40 nM | 200 nM | 1,000 nM | 200 nM | 1,000 nM |
| NK-4 | 1 | 152.5 | 158.0 | 164.3 | 30.5 | 27.7 |
| NK-5 | 2 | 150.0 | 151.8 | 84.0 | 23.1 | 41.7 |
| NK-6 | 7 | 105.0 | 117.0 | 141.9 | 32.6 | 41.5 |
| NK-36 | 3 | 123.0 | 115.5 | 137.2 | 33.8 | 28.7 |
| NK-44 | 5 | 193.4 | 154.3 | 78.5 | 29.3 | 22.9 |
| NK-1883 | 12 | 104.8 | 105.1 | 87.5 | 13.3 | 13.3 |
| NK-3134 | 13 | 103.9 | 119.6 | 122.8 | 29.4 | 18.2 |
| NK-4781 | 14 | 110.0 | 106.6 | 89.1 | 31.2 | 88.3 |
| NK-10089 | 15 | 110.6 | 123.4 | 91.6 | 0.0 | 29.3 |
| NK-10214 | 16 | 109.3 | 123.1 | 103.3 | 23.6 | 48.8 |
| NK-10358 | 17 | 119.4 | 163.5 | 138.6 | 2.0 | 35.7 |

<Neuronal growth acceleration activity>

As shown in Table 2, NK-5 (Chemical formula 2) showed a neurocyte growth acceleration activity equivalent to NK-4 at low (40 nM) and medium (200 nM) concentrations. On the other hand, NK-6 (Chemical formula 7) and NK-36 (Chemical formula 3) exhibited a slightly strong neurocyte growth acceleration activity from the high (1,000 nM) concentration region. This confirms that although the neurocyte growth acceleration activity is slightly weaker than that of NK-4 (Chemical formula 1), NK-6 (Chemical formula 7) and NK-36 (Chemical formula 3) also have a growth acceleration activity on cell lines of the nervous system as in NK-5 (Chemical formula 2). Regarding the neurocyte growth acceleration activity, NK-44 (Chemical formula 5) showed the strongest activity, which showed about 1.9 times as much cell proliferation as the negative control at the concentration of 40 nM. With respect to NK-44 (Chemical formula 5), as in NK-5 (Chemical formula 2), slight growth inhibition was observed in the high concentration range, indicating that the optimal concentration is lower than NK-4, i.e., the neurocyte growth acceleration activity of NK-44 is higher than NK-4. Regarding the neurocyte growth acceleration activity, it was confirmed that NK-10358 (Chemical formula 17) is a compound having the equivalent activity as NK-4. It was also confirmed that NK-10089 (Chemical formula 15), NK-10214 (Chemical formula 16) and NK-3134 (Chemical formula 13) are compounds having slightly weaker activity than NK-6 and NK-36. NK-1883 (Chemical formula 12) and NK-4781 (Chemical formula 14) were confirmed as compounds having almost no growth acceleration activity. All the compounds shown in the tables did not show remarkable cytotoxicity to PC-12HS cells in the concentration range tested.

<Neurite-outgrowth promoting activity>

As shown in Table 2, it was shown that 4 compounds, NK-5 (Chemical formula 2), NK-6 (Chemical formula 7), NK-4781 (Chemical formula 14) and NK-10214 (Chemical formula 16) have a stronger neurite-outgrowth promoting activity than NK-4 (Chemical formula 1). NK-36 (Chemical formula 3), NK-44 (Chemical formula 5) and NK-3134 (Chemical formula 13) have almost equivalent neurite-outgrowth promoting rate to NK-4. NK-4781 (Chemical formula 14) showed a very strong neurite-outgrowth promoting activity at high concentration (1,000 nM) but showed equivalent neurocyte growth acceleration activity to the negative control.

NK-4 (Chemical formula 1) is a compound carefully selected from a chemical compound library consist of 239 dye compounds, as a compound having both potent neurocyte growth acceleration activity and neurite-outgrowth promoting activity. Surprisingly, however, it was revealed that there are compounds having an activity comparable to NK-4 or significantly exceeding the activity of NK-4 from the group of low molecular weight dye compounds newly screened for this time. As compounds having both a neurocyte growth acceleration activity and a neurite-outgrowth promoting activity, NK-5 (Chemical formula 2), NK-44 (Chemical formula 5), NK-36 (Chemical formula 3), which are trimethine cyanine dye compounds having quinoline structures, and NK-6 (Chemical formula 7), which is a monomethine dye compound, are promising. In particular, it was shown that NK-5 (Chemical formula 2) and NK-44 (Chemical formula 5) can be used advantageously as agents that can be expected to have an activity higher than NK-4, and from the viewpoint of chemical structure, it has been inferred that the basic structure in which the 4th position of two quinoline structures are linked by a methine chain is likely to be used advantageously.

<Experiment 1-3: Effect on intracellular ROS production>

From the above-mentioned Experiment 1-1 and Experiment 1-2, it was shown that NK-5 (Chemical formula 2), NK-5 analogs (Chemical formula 3 and Chemical formula 5) and NK-6 (Chemical formula 7) have strong neurocyte growth acceleration activity and neurite-outgrowth promoting activity, and therefore, it was verified by the following experiment whether these compounds also act effectively on oxidative damage to neurocyte. That is, PC12-HS cells were oxidatively stimulated with hydrogen peroxide, and the intracellular ROS generated at that time was quantified using a fluorescent reagent, and the inhibitory effect of the dye compounds on intercellular ROS production, when an external oxidative stress was applied, was evaluated.

Similar to Experiment 1-1 and Experiment 1-2, precultured PC12-HS cells were cultured for 24 hours with test compounds diluted in culture medium at final concentrations of 125, 250, 500 and 1000 nM. Thereafter, the culture supernatant was removed, the cells were washed with Hank's buffer (60 $\mu$M $CaCl_2$, 400 $\mu$M $MgSO_4$, 500 $\mu$M $MgCl_2$), and then 100 $\mu$l of ROS detection reagent CM-$H_2$DCFDA (commercialized by FUJIFILM Wako Pure Chemical Corporation, Tokyo, Japan) adjusted to 5 $\mu$M with the same buffer, was added to each well. The PC12-HS cells were cultured at 37 °C for 30 minutes to allow the cells to take up CM-$H_2$DCFDA, and then, the solution containing CM-$H_2$DCFDA was removed. Then, the cells were washed with Hank's buffer, and then added 50 $\mu$M of hydrogen peroxide (commercialized by FUJIFILM Wako Pure Chemical Corporation, Tokyo, Japan) diluted with Hank's buffer in a volume 100 $\mu$l/well and stimulated at 37 °C for 2 hours. The solution containing hydrogen peroxide was removed, then an 200 $\mu$l of Hank's buffer was added to each well, and the cells were measured for fluorescent intensity at a wavelength of 492-527 nm, which is

an index of intracellular ROS production, by a fluorescence microplate reader. In addition, after measuring the fluorescence intensity, the cells were fixed by adding 20 μl of glutaraldehyde to each well, and the amount of methylene blue dye uptake was measured as an absorbance at 650 nm, according to a conventional method, the result was used as an index of the number of living cells. The amount of ROS production per cell was indicated by the value that fluorescence intensity (492-527 nm) was divided by the absorbance at 650 nm in each well. The amount of ROS production per cell calculated from cells to which only hydrogen peroxide was added without containing the test compound as a negative control, was set as 100%, and the relative value when the test compound was added was determined, and is shown in Table 3, as the ROS production rate (%) per cell of each well. Measurements were performed for each concentration of each test compound in triplicate, and the mean values were described.

Table 3

| Compound (NK No.) | Chemical formula No. | Intercellular ROS production (%) | | | |
|---|---|---|---|---|---|
| | | 125 nM | 250 nM | 500 nM | 1,000 nM |
| NK-4 | 1 | 126.5 | 113.2 | 132.1 | 110.5 |
| NK-5 | 2 | 66.4 | 65.1 | 40.5 | 19.6 |
| NK-6 | 7 | 108.6 | 81.2 | 69.8 | 66.2 |
| NK-36 | 3 | 88 | 43.5 | 51 | 8.3 |
| NK-44 | 5 | 75.4 | 65.1 | 34.1 | 27.1 |

As shown in Table 3, NK-4 (Chemical formula 1) showed no inhibitory effect on intercellular ROS production, at concentration rage of 125 to 1,000 nM. On the other hand, NK-5 (Chemical formula 2), NK-6 (Chemical formula 7), NK-36 (Chemical formula 3), and NK-44 (Chemical formula 5) suppressed the intercellular ROS production induced by hydrogen peroxide stimulation, in a concentration-dependent manner. In particular, the effect of NK-5 and NK-36 was strong and more remarkable than that of NK-44 and NK-6.

It is already known that NK-4 (Chemical formula 1) has radical-scavenging ability, but as described above, the same compound did not affect intracellular ROS production. The detailed reason for this is unknow, but unlike NK-5 (Chemical formula 2) and NK-6 (Chemical formula 7), it is considered that one of the reasons is that the amount required for activity expression could not be transferred into the cells. This suggests a problem in drug utilization of cyanine dye compounds represented by General formulae 1 to 3 including NK-4, since these compounds have a heterocyclic or heteroaromatic trinuclear structure, it is considered that they inevitably have a large molecular weight, become bulky, and they are disadvantageous in permeability to cells and tissues. On the contrary, the compounds that showed strong activity in Experiment 1-3 were chemically smaller molecules than NK-4, and the experimental findings tell that these compounds have good cellular permeability.

As shown in Experiment 1-1 and Experiment 1-2, NK-5, its analogs (NK-36, NK-44), and NK-6 do not show cytotoxicity as NK-4, and have a strong neurocyte growth accelerating activity and neurite-outgrowth promoting activity at nanomolar concentrations. On the other hand, In Experiment 1-3, it was shown that NK-5, its analogs (NK-36, NK-44), and NK-6 also have an action that NK-4 does not have, that is, an action that suppresses the production of intracellular ROS, and therefore, it has been shown that NK-5 (Chemical formula 2), its analogs (NK-36 (Chemical formula 3), NK-44 (Chemical formula 5)), and NK-6 (Chemical formula 7) could become anti-neurodegenerative disease agent having novel properties.

<Experiment 2: Brain Transferability of Dye Compounds>

In Experiment 1 described above, it was inferred that NK-5 (Chemical formula 2) and its analogs (NK-36 (Chemical formula 3), NK-44 (Chemical formula 5)) and NK-6 (Chemical formula 7) have strong neuronal cell proliferation promoting action and neurite outgrowth action, and had good cell permeability. In order to verify this, the physicochemical characteristic indicators related to the blood-brain barrier permeability of these dye compounds were examined based on predictions from experimental and theoretical calculations.

<Experiment 2-1: Measurement of LogP of dye compounds>

As a pharmacological index indicating the brain transferability of a compound, LogBB representing the permeability to brain tissue and LogPS representing the permeation rate of the blood-brain barrier (hereinafter referred to as "BBB") are known. When simulating these parameters by chemical calculations, machine-learning is required by inputting

some actually measured parameters, but as described in, for example, "Therapeutic Delivery", Vol. 6 (No. 7), pp. 961 to 971 (2015), the most influential measured parameter is the octanol/water partition coefficient called LogP, and it is said that the larger LogP, the better the lipid solubility, and the higher the permeability to brain tissue. Therefore, the LogP values of NK-5 and NK-6 were measured and compared with those of NK-4.

Measurements of octanol/water partition coefficients (hereinafter referred to as "LogP") were carried out according to "Analytical Chemistry (BUNSEKI KAGAKU)", Vol. 53 (No. 9), p. 953-958 (2004), and "Japanese Industrial Standards", Z7260-107 (2000), Measurement of partition coefficients (1-octanol/water)-Flask shaking method." That is, an appropriate amount of the test compound was added to 1-octanol (for measuring the partition coefficient, commercialized by Kanto Chemical Co., Inc.) to prepare a saturated 1-octanol solution for each sample. 1 ml of this solution was mixed with 1 ml of ultrapure water, and shaken at 25 °C using a shaker at a rate of 20 revolutions per minute for 5 minutes. The mixed solution was centrifuged at 15,000 rpm for 15 minutes at 25 ° C using a centrifuge, and after confirming that the 1-octanol layer and the aqueous layer were completely separated from each other, both layers were collected in 500 μl portions while preventing contamination from each other layer. The concentration of the test substance in the recovered solution was quantified by the HPLC method shown below, and the partition coefficient was calculated from the concentration of the test substance in the 1-octanol layer ([C] o) and the concentration of the test substance in the water layer ([C] w) by the following formula, and shown in Table 4.

Formula 1:

$$\text{partition coefficient} \quad \text{LogP} = \text{Log} \frac{[C]o}{[C]w}$$

[C] o: Concentration of test sample in 1-octanol layer (mol/L)

[C] w: Concentration of test sample in water layer (mol/L)

<Preparation of standard solution for calibration curve>

20 mg of each test compound was precisely weighed and dissolved in 50 ml of mobile phase to prepare a 400 μg/mL solution. This solution was further diluted with a mobile phase to prepare standard solutions of 5,000, 2,500, 1,000, 500, 250, 100 and 50 ng/mL.

<Preparation of sample solution>

2,500 μl of mobile phase was added to the collected sample solution (500 μl each), mixed well, and then filtered through a 0.45 μm organic solvent resistant filter to obtain a sample solution, and 10 μl of each sample was injected into HPLC and analyzed.

<HPLC analysis conditions (NK-4)>

HPLC system: HPLC Prominence LC-20AD (Shimadzu)
Detector: Ultraviolet spectrophotometer SPD-M20A (Shimadzu)
Column (NK-4): XBridgeC8 5μm (Waters)
Column diameter x Column length: 4.6 x 250 mm
Column temperature: 40 °C
Mobile phase: Water/Methanol/Acetic acid = 60/40/0.1 by volume
Flow rate: 0.7 mL/min
Detection wavelength: 776 nm
Quantitative range: 50 -5,000 ng/L

<HPLC analysis conditions (NK-5 / NK-6)>

Conditions different from those of NK-4 are described below.

Column: COSMOSIL 5C8-AR-300 (manufactured by Nacalai Tesque) Mobile phase: Acetonitrile/Triethylamine/Acetic acid = 70/30/0.1 by volume Detection wavelength: 700 nm (NK-5), 588 nm (NK-6)

Table 4

| Compound (NK No.) | Chemical formula No. | Concentration (μg/mL) | | partition coefficient | |
|---|---|---|---|---|---|
| | | octanol layer [C]o | Water layer [C]w | P [C]o / [C]w | LogP |
| NK-4 | 1 | 1.27 | 13.31 | 0.095 | -1.02 |
| NK-5 | 2 | 46.99 | 15.2 | 3.09 | 0.49 |
| NK-6 | 7 | 7.99 | 16.96 | 0.47 | -0.33 |

As shown in Table 4, the compound concentrations distributed to the 1-octanol layer were in the order of NK-5 > NK-6 > NK-4, and the partition coefficient LogP was also in the same order. This suggests that the relative lipophilicity between the compounds, and hence the permeability to tissues and cells, is also in this order. Therefore, as shown in Experiment 1-3, it is also supported by the simulation based on computational chemistry that NK-5 and its analogs (NK-36, NK-44) and NK-6 have excellent transferability into cells. Especially with respect to NK-5, the P value was 30 times higher than that of NK-4, and it was considered that the transferability of NK-5 reaches the level at which oral administration is possible, which has been difficult until now. The experimental findings show that NK-5 (Chemical formula 2) and NK-6 (Chemical formula 7) have a significant advantage over NK-4 (Chemical formula 1) in terms of tissue permeability.

<Experiment 2-2: Prediction of brain permeability of dye compounds by computational chemistry>

As described above, as a pharmacological index indicating the brain transferability of a compound, a LogBB representing penetration into brain tissue and a LogPS representing BBB permeation rate are known. These parameters can be obtained experimentally using so-called "living organisms" such as experimental animals, but the experimental procedure is difficult, very expensive and low throughput. In recent years, it has become possible to make computer predictions of these parameters using computational chemistry techniques. For example, the simulations described in "Drug Metabolism and Disposition", Vol. 32 (No. 1), pp. 132-139. (2004), "Journal of Pharmaceutical Science", Vol. 98 (No. 1), pp. 122-134 (2009) and "Therapeutic Delivery", Vol. 6 (No. 7), pp. 961-971 (2015), have steadily improved the prediction accuracy. Therefore, in order to estimate the relative brain transferability and brain permeability of the dye compounds in more detail, seven compounds represented by Chemical formulae 1, 2, 5 to 7, 10 and 11 were selected to calculate LogP (hereinafter, referred to as "cLogP" to distinguish it from the actually measured value of LogP) and LogBB. All these seven compounds have iodine as a counter anion.

Similar to the LogP, the larger the value of cLogP, the more excellent the lipophilicity and the higher the permeability to the brain tissue. As a more specific numerical range, for example, it is preferable in brain transferability that cLogP is in the range of 1 to 3, and optimal when it is around 2, as a rule of thumb in the drug discovery of the central nervous system drugs as described in "Pharmacia", Vol. 48 (No. 8) 761 to 766 (2012), and the like. On the other hand, LogBB is a partition coefficient expressed by the following equation in principle, and the larger the value, the higher the permeability from blood vessels to brain tissue. "ADMEWORKS (registered trademark) software" (commercialized by Fujitsu Kyushu Systems Limited) was used for computer simulations. The calculated cLogP and LogBB of each dye compound are shown in Table 5. In the simulation of each physical property value, the calculation was performed after structural corrections based on the assumption that the cationized nitrogen of the target compound and the iodine ion were covalently bonded.

Formula 2:

$$\text{partition coefficient} \quad \mathrm{LogBB} = \mathrm{Log}\,\frac{[\mathrm{C}]_{\mathrm{brain}}}{[\mathrm{C}]_{\mathrm{blood}}}$$

[C]brain : Concentration of test sample in brain

[C]blood : Concentration of test sample in blood

Table 5

| Compound (NK No.) | Chemical formula No. | Value from simulation | |
|---|---|---|---|
| | | cLogP | LogBB |
| NK-4 | 1 | 0.0108 | 2.7820 |
| NK-5 | 2 | 2.2964 | 2.9833 |
| NK-44 | 5 | 3.1930 | 3.3882 |
| NK-45 | 6 | 3.6281 | 3.2699 |
| NK-6 | 7 | 1.8953 | 3.1310 |
| NK-1486 | 11 | 3.2550 | 3.5308 |
| NK-10745 | 10 | 2.8113 | 3.4940 |

As shown in Table 5, when the three compounds of NK-4 (Chemical formula 1), NK-5 (Chemical formula 2), and NK-6 (Chemical formula 7) were compared relative to each other, the cLogP value was in the order of NK-5 > NK-6 > NK-4, the same tendency as the actually measured LogP values in Table 4 was shown. Other than these three compounds, compounds having an n-butyl group or an isopentyl group as the N-alkyl group added to the quinoline ring (Chemical formulae 5, 6, 10 and 11) showed high cLogP values. As described above, the preferable cLogP value as a central nervous system drug is said to be 1 to 3, and the cLogP value of the test compounds other than NK-4 is generally within that range.

As also shown in Table 5, regarding the LogBB value, the numerical difference between the compounds was not as large as that of cLogP. It was inferred that NK-4 (Chemical formula 1) has the lowest permeability to the brain among the test compounds. As in the case of cLogP, it was found that compounds having n-butyl or isopentyl groups as side chain alkyl side chains (Chemical formulae 5, 6, 10 and 11) exhibited high values in LogBB, and in the quinoline-based dinuclear monomethine and trimethine cyanine dyes, the carbon chain length of the alkyl side chain bonded to the 1st position of nitrogen atom of quinoline was shown to be related to brain permeability. Alternative evaluation methods based on computational chemistry have already been used as a general-purpose tool in the field of drug discovery, so their reliability is high, and the above calculation results indicate that these compounds have excellent potential as central system drugs.

<Experiment 3: Effect of dye compounds on free radicals>

In ischemic diseases typified by myocardial infarction and cerebral infarction, ROS is largely involved in tissue damage during blood reperfusion after ischemia. In particular, brain tissue is known to be highly susceptible to oxidative damage caused by hydroxyl radicals and peroxyl radicals due to its high lipid content, and brain function is impaired by direct and/or indirect neuronal degeneration caused by these free radicals. Therefore, to know whether the dye compound which is the active ingredient of the anti-neurodegenerative disease agent of the present invention effectively scavenges free radicals, four types of model radicals were selected, and the scavenging activity to them was measured by absorbance method and electrons spin resonance method (hereinafter, abbreviated as "ESR").

<Test samples>

In this experiment, NK-4 (Chemical formula 1), NK-5 (Chemical formula 2) and NK-6 (Chemical formula 7) were selected from the dye compounds used in Experiment 1 to compare intensities of the effect. In Experiments 3-2 to 3-4, L-ascorbic acid having an antioxidant ability was used as a positive control.

<Experiment 3-1: Measurement of DPPH radical scavenging ability>

2,2-Diphenyl-1-picrylhydrazyl (hereinafter referred to as "DPPH") is a radical with an unpaired electron and has

the oxidizing ability of other substances. In addition, because it is stable and easy to handle, it is widely used as an artificial radical-like substance for measuring the antioxidant ability of antioxidants. When DPPH reacts with a substance having antioxidant ability, it loses its own oxidizing ability and its color changes from deep purple to colorless. Therefore, the DPPH scavenging ability of the dye compounds was examined by measuring the disappearance of the deep purple color exhibited by the DPPH by the absorbance method.

<Test samples>

NK-4 (Chemical formula 1), NK-5 (Chemical formula 2) and NK-6 (Chemical formula 7) were used as test compounds, and the test samples were prepared in the same manner as described in Experiment 1-1. Only in Experiment 3-1 (measurement of DPPH radical scavenging ability), NKX-4076 represented by Chemical formula 43 was synthesized and added as a test compound in order to confirm the correlation between the structure and activity of the dye compound.

[Chem. 14]

Chemical formula 43:

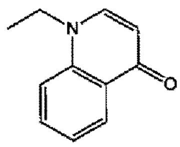

DPPH (commercialized by Tokyo Kasei Kogyo Co., Ltd., Tokyo, Japan) was dissolved in ethanol to prepare a 167 μM DPPH ethanol solution, and 90 μl of the solution was added to each well of the 96-well microplate. A 60 μl each of the test compound serially diluted with ethanol was added to the DPPH ethanol solution, and the mixture was reacted at room temperature for 2 hours, and then the absorbance at 490 nm was measured. The absorbance of the well to which the test compound was not added, was defined as 100% radical intensity, and the radical intensity of the well containing the test compound at each concentration was showed as its relative percentage (%) to the control. From those value, the 50% inhibitory concentration of the DPPH radical (Hereinafter, it is referred to as "$IC_{50}$") was calculated. Measurements were performed triplicate for each concentration of each test compound, and the mean value is shown in Table 6. Table 6 also shows the evaluation results of superoxide scavenging ability, hydroxyl radical scavenging ability, and peroxyl radical scavenging ability, which will be described later.

As shown in Table 6, NK-4 (Chemical formula 1), NK-5 (Chemical formula 2) and NK-6 (Chemical formula 7) all exhibited DPPH radical scavenging ability, and the intensity thereof was in the order of NK-5 > NK-6 > NK-4. Although not shown in Table 6, the $IC_{50}$ value of NKX-4076 represented by Chemical formula 43 is calculated to be 10,000 μM or higher, indicating that the compound having a quinoline structure has substantially no DPPH radical-scavenging ability. From this, it was inferred that the structural unit contributing to DPPH radical-scavenging activity of these compounds was not the heteroaromatic ring itself but the methine structure which connect the heteroaromatic rings. On the other hand, the relative activity based on the $IC_{50}$ value is about 4 times that of NK-4 for NK-5 and about 2 times that of NK-4 for NK-6 and this suggests that the scavenging ability of DPPH radicals is due to the presence of a methine chain in the molecule. It is an essential requirement, however, the scavenging ability of DPPH radicals did not correlate with the length of the methine chain connecting the quinoline rings, and it is presumed that the straight-chain methine structure having no branches works preferably.

<Experiment 3-2: Measurement of superoxide scavenging ability>

Superoxide is a ROS with three electrons in two π* orbitals (antibonding π orbitals) of the molecular orbital of an oxygen molecule, and has radical reactivity and anionic properties. It is produced by one-electron reduction of oxygen molecule, and in vivo, produced by mitochondrial respiratory chain electron transmission system, xanthine oxidase, and NADH oxidase, etc., then scavenged by superoxide dismutase (hereinafter referred to as "SOD"). In this experiment, superoxide generated by reacting hypoxanthine with xanthine oxidase was captured by 5,5-dimethyl-4-phenyl-1-pyrroline N-oxide (hereinafter referred to as "4PDMPO") as a spin trapping agent, and SOD-like activity by the test compound was measured by ESR.

Specifically, A 50μl of aqueous solution of hypoxanthine (commercialized by Fuji Film Wako Pure Chemical Industries, Ltd., Tokyo, Japan) prepared to 1mM was added to a glass test tube. Then, 30μl of DMSO and 50μl of 4.5 M 4PDMPO (commercialized by Fuji Film Wako Pure Chemical Industries, Ltd.) were added to the tube and stirred, and 50μl of the test compound solution was further added, and then 50μl of 0.4 unit/mL xanthine oxidase solution (commercialized by Roche) was added and mixed by vortex for 10 seconds. The ESR spectrum of the reaction mixture was

measured 40 seconds after the addition of xanthine oxidase, and the radical scavenging ability of the test compound was shown as its relative percentage thereof, with the case where no dye compound was added as the radical intensity of 100%. Measurements were performed in triplicate for each of the concentrations of each test compound, and the $IC_{50}$ was calculated using the average value thereof and shown in Table 6. Ascorbic acid (commercialized by Fuji Film Wako Pure Chemical Industries, Ltd.), which is known as an antioxidant, was used as a positive control. The ESR measurement conditions at this time will be described later.

As shown in Table 6, all the test compounds had the ability to scavenge superoxide radicals, and NK-6 had the same ability as ascorbic acid known as a strong radical scavenger. When comparing $IC_{50}$ values of the test compounds, NK-5 and NK-6 showed a superoxide radical scavenging ability more than twice that of NK-4, and the relative intensities of the scavenging activities of these three compounds were almost the same as those of the scavenging ability for DPPH radicals.

<Experiment 3-3: Measurement of hydroxyl radical scavenging ability>

Hydroxyl radical is a kind of free radical, and is known to have extremely high reactivity in vivo, i.e., highly tissue-damaging. Hydroxyl radicals are produced in vivo by exposure of hydrogen peroxide to ultraviolet rays and by the reaction with hydrogen peroxide with a divalent iron compounds (Fenton's Reaction), and are scavenged by antioxidants such as beta carotene, vitamin E, uric acid, linoleic acid, cysteine, flavonoids, and glutathione. In this experiment, hydroxyl radicals generating from diethylenetriamine-N, N, N', N'-pentaacetic acid (DTPA) by Fenton reaction were trapped with 4PDMPO and measured by ESRs.

Specifically, each of the dye compounds dissolved in DMSO was diluted with purified water to obtain a test compound solution. To a glass test tube, 50 μl of 1 mM hydrogen peroxide solution and 50 μl of 356 mM 4PDMPO were added, followed by 50 μl of the test compound solution. Then, 50 μl of an aqueous solution containing 1 mM $FeSO_4$ and 1 mM DTPA (commercialized by Fuji Film Wako Pure Chemical Industries, Ltd.) was added thereto, mixed with a vortex mixer for 10 seconds, and then reacted for exactly 40 seconds, and the reaction solution was used for ESR measurement. The radical scavenging ability of the test compound was shown as a relative percentage thereof, with the case where no dye compound was added as the radical intensity of 100%. Measurements were performed in triplicate for each of the concentrations of each test compound, and their mean values were used to calculate $IC_{50}$, which is also shown in Table 6. For positive control, L-ascorbic acid was used as in the case of Experiment 3-2. The ESR measurement conditions in this experiment will be described later.

As shown in Table 6, all of the test compounds had the ability to scavenge hydroxyl radicals, and when $IC_{50}$ was used as an intensity index of hydroxyl radical scavenging ability, NK-5 (Chemical formula 2) showed the strongest scavenging activity, followed by NK-4 (Chemical formula 1) and NK-6 (Chemical formula 7). Although ascorbic acid, known as a potent natural antioxidant, was used as a control in this experiment, NK-4 and NK-5 were shown to have markedly higher activity than L-ascorbic acid. NK-6 also had hydroxyl radical scavenging ability, although that was inferior to ascorbic acid.

<Experiment 3-4: Measurement of peroxyl radical scavenging ability>

Peroxyl radical, a kind of free radical, is also a kind of in vivo lipid peroxides produced by the reaction of unsaturated fatty acid and hydroxyl radical. Organs with high lipid content, such as the brain, are known to be highly susceptible to peroxyl radicals, and the effect of scavenging these radicals is extremely important in neurodegenerative diseases in which oxidative stress is involved in pathogenesis. Since the peroxyl radical generated by heating of 2,2'-azobis (2-aminodipropane) dihydrochloride (hereinafter referred to as "AAPH") is a model of biologically derived radicals, in this experiment, the ability of the test compound to scavenge the peroxyl radical was measured by ESR.

Specifically, each of the dye compounds dissolved in DMSO was diluted with 0.1 M phosphate buffer to prepare a test compound solution. To 50μl of 0.1 M phosphate buffer (pH7.4), 50 μl of 720 mM DMPO, 50 μl of the test compound solution, and 50 μl of 100 mM AAPH (commercialized by Fuji Film Wako Pure Chemical Industries, Ltd.) were added, mixed with a vortex mixer for 10 seconds, and then mixed solution was reacted in a constant temperature bath at 37 °C for 2 minutes and 50 seconds, and the reaction solution was used for ESR measurement 30 seconds after completion of the reaction. The radical scavenging ability of the test compound was presented as its relative percentage thereof, with the case where no dye compound was added as the radical intensity of 100%. Measurements were performed in triplicate for each of the concentrations of each test compound, and the $IC_{50}$ was calculated using the mean value thereof and shown in Table 6. L-ascorbic acid was used as a positive control in the same manner as in Experiments 3-2 and 3-3. The ESR measurement conditions in this experiment will be described later.

Table 6

| Compound (NK No.) | Chemical formula No. | $IC_{50}(\mu m)$ | | | |
|---|---|---|---|---|---|
| | | DPPH radical | superoxide radical | hydroxyl radical | peroxyl radical |
| ascorbic acid | - | not done | 66 | 38 | 32 |
| NK-4 | 1 | 772 | 163 | 9.4 | 6.4 |
| NK-5 | 2 | 182 | 42 | 3.4 | 2.9 |
| NK-6 | 7 | 380 | 69 | 62 | 43 |

As shown in Table 6, NK-4 (Chemical formula 1), NK-5 (Chemical formula 2) and NK-6 (Chemical formula 7) all had the ability to scavenge peroxyl radicals. When $IC_{50}$ value is considered as a radical scavenging ability index, NK-5 showed the strongest scavenging activity, followed by NK-4 and NK-6, and this tendency correlated with the scavenging ability of hydroxyl radicals.

<ESR measurement method>

Detailed conditions of the ESR measurement are described in this section. A reaction solution for measuring various radicals was placed in a flat quartz cell for ESR, and the ESR spectrum was measured by an ESR device (commercialized by JEOL Ltd., trade name "Free radical Controller JES-FR30"). When measuring the ESR spectrum derived from each radical, instead of the test compound solution, purified water was mixed with the reaction solution in the case of superoxide and hydroxyl radical measurement, and 0.1 M phosphate buffer was mixed with the reaction solution in the case of peroxyl radical measurement. It was mixed to react in the same manner as in the case of measuring the test compound. The peak height of the ESR spectrum corresponding to each radical at this time was set as 100, and the relative intensity when the solution was added and reacted was obtained and expressed as the radical residual rate (%). The ESR measurement parameters in this experiment were set as follows.

<Measurement conditions>

| | |
|---|---|
| Power: | 4 mW |
| Magnetic field: | 335.5 mT |
| Sweep time: | 2 min |
| Modulation width: | 0.079 mT |
| Amplitude: | 79 (in the case of hydroxyl radical measurement) |
| | 125 (in the case of peroxyl radical measurement) |
| | 250 (in the case of superoxide radical measurement) |
| Time constant: | 0.1 sec |
| Accumuration: | 1 |

As shown in Experiment 3 described above, NK-5 (Chemical formula 2) scavenges hydroxyl radicals and peroxyl radicals more strongly than NK-4 (Chemical formula 1), and therefore, administration of NK-5 to living organisms is expected to remarkably suppress oxidative stress caused by hydroxyl radicals and/or peroxyl radicals generated during blood reperfusion in ischemic diseases. From the results of Experiment 1-1 and Experiment 1-2, NK-5 (Chemical formula 2) and NK-6 (Chemical formula 7) are expected to suppress neuronal cell death since they both have the neurocyte growth accelerating activity and the neurite outgrowth promoting activity equivalent to NK-4 or higher than that of NK-4 (Chemical formula 1). In addition, in Experiments 1-3, NK-5 (Chemical formula 2), its analogs, and NK-6 (Chemical formula 7) effectively inhibited ROS production in neurocyte induced by oxidative stress, suggesting that these compounds are effective not only as anti-neurodegenerative agents but also as brain protectants, radical scavengers, oxidative stress inhibitors, lipid peroxide production inhibitors, and brain oxidative damage inhibitors. Further, the radical scavenging action of NK-5 (Chemical formula 2) shown in Experiment 3 was remarkable, thus it showed a stronger scavenging ability than that of NK-4 (Chemical formula 1) in all four radicals subjected to the experiment. On the other hand, NK-6 (Chemical formula 7) was shown to have a strong action on DPPH radicals and superoxide. This indicates that NK-5 and NK-6 have different specificities regarding radical scavenging, suggesting that the combination

of these two compounds can be designed to effectively scavenge ROSs including a larger variety of radicals.

<Experiment 4: Solubilities of compounds>

It is considered that NK-4 (Chemical formula 1; molecular weight: 789.54), one of the pentamethine cyanine dyes having quinoline trinuclear structures, disclosed in a prior art (Patent Literature 6), has a relatively large molecular mass and therefore, a low solubility in comparison with general central nervous system drugs. Accordingly, solubilities in water and ethanol of NK-5 (Chemical formula 2; molecular weight: 480.38) and NK-6 (Chemical formula 7; molecular weight: 454.34), both having neurocyte growth accelerating action, neurite-outgrowth promoting action, and radical scavenging action as NK-4, were evaluated.

<Method for quantifying compounds (absorbance method)>

A test sample solution, prepared by dissolving each test compound in DMSO to give a concentration of 5 mg/mL, was diluted with 70% (v/v) acetonitrile aqueous solution, and the absorption spectrum at the wavelength range of 400 nm to 900 nm is measured by a spectrophotometer ("FLEX STATION 3", manufactured by Molecular Devices Japan, Tokyo, Japan). The maximum absorption wavelength of each test compound was determined, then the correlation equation between the absorbance and the concentration at the wavelength was calculated to prepare a calibration curve, and then the following quantitative test was carried out. The maximum absorption wavelengths of NK-4, NK-5, and NK-6 in the 70% (v/v) acetonitrile aqueous solution were 770 nm, 704 nm, and 588 nm, respectively, and the concentration dependence at the maximum absorption wavelength was confirmed for each compound.

The solubility of each dye compound was measured as follows with reference to the method described in the Japanese Pharmacopoeia. Each test compound was dried under reduced pressure at 120°C for 2 hours using a vacuum dryer ("VACUUME DRY OVEN DP41", manufactured by Yamato Scientific Co., Ltd., Tokyo, Japan), weighed 20 mg each, and added 100 mL of distilled water. Then, the resulting mixture was stirred at room temperature under a dark condition. After 30 min, 1 mL of the mixture was sampled at 3 times from each test solution, centrifuged at 18,400 × g for 5 min, 0.3 mL of the supernatant was collected and admixed with 0.7 mL of acetonitrile to make a total volume of 1.0 mL. Then, the absorbance was measured at the maximum absorption wavelength corresponding to each of the dyes described above, and the concentration of dye was calculated from the calibration curve.

Table 7

| Compound (NK No.) | Chemical Formula No. | Solubility (mg/L) | |
|---|---|---|---|
| | | Distilled water | Ethanol |
| NK-4 | 1 | 5 | 46 |
| NK-5 | 2 | 35 | 1,036 |
| NK-6 | 7 | 93 | 645 |

As shown in Table 7, it was found that the solubility of each dye compound in distilled water was highest in NK-6 (Chemical formula 7), followed by NK-5 (Chemical formula 2), and the water solubility of both compounds was significantly higher than that of NK-4 (Chemical formula 1). Regarding the solubility in ethanol, the order of NK-5 and NK-6 was reversed, but both were shown to have significantly higher solubility than NK-4. These results indicate that NK-5 and NK-6 are significantly more soluble than NK-4, and therefore can be used extremely advantageously in terms of bioavailability and drug availability.

From the series of results in Experiments 1 to 4, it is clear that NK-5, NK-6 and their analogs having different alkyl side chains (Chemical formulae 2 to 11), those are the compounds represented by General formula 4, have remarkable advantages in terms of their effect, formulation suitability, and brain permeability in comparison with NK-4 (Chemical formula 1), a dye compound has been proposed as an active ingredient of a neurodegenerative disease agent.

The following examples explain the anti-neurodegenerative disease agent of the present invention, but they do never restrict the present invention.

Example 1

<Agent for oral ingestion>

NK-5 (compound represented by Chemical formula 2) and NK-6 (compound represented by Chemical formula

7), both manufactured by Hayashibara Co., Ltd., are mixed in equal molar amounts, and made into fine powder using a mortar. To 5.0 parts by mass of the resulting mixed power, 3.75 parts by mass of sodium hydrogen carbonate, 1.5 parts by mass of hydrous crystalline α,α-trehalose ("TREHALOSE 100PH", commercialized by Hayashibara Co., Ltd., Okayama, Japan), 0.25 part by mass of magnesium stearate were added and mixed homogeneously, and 0.5 g each was made into a tablet by a conventional method. The product can be used as an anti-neurodegenerative disease agent for oral administration, which has high and lasting effects. Further, the product can also be used as a neurodegenerative inhibitor, a neuronal protective agent, a neurite promoter, and a therapeutic agent for pathological conditions and neurological dysfunction associated with neurodegeneration. Furthermore, the product can be used as a brain protective agent, inhibitor of a brain oxidative injury, inhibitor of an ischemic cerebral injury, inhibitor of a cerebral infarction lesion progression, a cerebral edema inhibitor, a late-onset neuronal death inhibitor, a brain function normalizing agent, oxidative stress inhibitor, anti-ulcer agent, hyperglycemia inhibitor, prophylactic/therapeutic agent for ocular diseases, organ preservative for transplantation, transplanted tissue/organ necrosis inhibitor, prophylactic/therapeutic agent for tissue/organ damage, prophylactic/therapeutic agent for radiation disorders, antitumor agents, tumor metastasis inhibitors, cytotoxic marker inhibitors, prophylactic/therapeutic agent for inflammatory diseases and associated tissue disorders, inhibitors for sensory cell, sensory nerve or sensory organ disorders, prophylactic/therapeutic agent for drug addiction, Ca/Na exchange inhibitor, prophylactic/therapeutic agents for pain and itching, protein kinase stimulants, prophylactic/therapeutic agent for mitochondrial encephalomyopathy, prophylactic/therapeutic agent for arterial obstruction/stenosis, inhibitor of blood-brain barrier disruption, therapeutic agent for drug addiction, an apoptosis inhibitor, inhibitor of a lipid peroxide production, a radical scavenger, an amyloid β peptide aggregation inhibitor, and an amyloid β peptide injury inhibitor. In addition, the anti-neurodegenerative disease agent of the present invention can also be used as a therapeutic or prophylactic agent for animals other than humans, including pets that have developed a neurodegenerative disease.

Example 2

<Agent for oral ingestion>

NK-5 and its analogs (NK-36, NK-37, NK-44 and NK-45), represented by Chemical formulae 2 to 6; and NK-6 and its analogs (NK-1946, NK-1945, NK-10745 and NK-1486), represented by Chemical formulae 7 to 11; (all manufactured by Hayashibara Co., Ltd., Okayama, Japan) were pulverized separately in advance. To 5.0 parts by mass of each compound, 3.75 parts by mass of sodium hydrogen carbonate, 1.5 parts by mass of ascorbic acid 2-glucoside (reagent grade "L-ascorbic acid 2-glucoside, commercialized by Hayashibara Co., Ltd., Okayama, Japan) and 0.25 parts by mass of magnesium stearate were added and mixed homogenously. Then, 0.5 g of the resulting mixture was made into a tablet by a conventional method. The product can be used as an anti-neurodegenerative disease agent for oral administration as in the case of Example 1 in the scope of application of the example. Further, the product has excellent storage stability due to the action of L-ascorbic acid 2-glucoside as a stable vitamin C, has no side effects even when ingested for a long period of time, and can be used safely by non-sick (presymptomatic) people.

Example 3

<Liquid agent for injection>

A solution prepared by dissolving 60 g of purified maltose for injection (manufactured by Hayashibara Co., Ltd., Okayama, Japan) in 370 g of purified water for injection; and another solution prepared by dissolving 2 g of lecithin and 120 mg of a compound selected from NK-5 and its analogs, represented by Chemical formulae 2 to 6 (NK-36, NK-37, KK-44, and NK-45), and 120 mg of a compound selected from NK-6 and its analogs, represented by Chemical formulae 7 to 11 (NK-1946, NK-1945, NK-10745, and NK-1486) (all manufactured by Hayashibara Co., Ltd., Okayama, Japan) in 170 g of purified water for injection; were mixed. The resulting mixture was sterilized by filtering and bubbled aseptic nitrogen gas to give a concentration of dissolved oxygen of about 0.1 ppm. One milliliter each of the resulting solution was dispensed into brown ampoules, and the ampoules were sealed under a nitrogen stream. The products are pyrogen-free and can be used as an anti-neurodegenerative disease agent for injection. Furthermore, the product can be used as a brain protective agent, inhibitor of a brain oxidative injury, inhibitor of an ischemic cerebral injury, inhibitor of a cerebral infarction lesion progression, a cerebral edema inhibitor, a late-onset neuronal death inhibitor, a brain function normalizing agent, oxidative stress inhibitor, anti-ulcer agent, hyperglycemia inhibitor, prophylactic/therapeutic agent for ocular diseases, organ preservative for transplantation, transplanted tissue/organ necrosis inhibitor, prophylactic/therapeutic agent for tissue/organ damage, prophylactic/therapeutic agent for radiation disorders, antitumor agents, tumor metastasis inhibitors, cytotoxic marker inhibitors, prophylactic/therapeutic agent for inflammatory diseases and associated tissue disorders, inhibitors for sensory cell, sensory nerve or sensory organ disorders, prophylactic/therapeutic agent for drug addiction, Ca/Na exchange inhibitor, prophylactic/therapeutic agents for pain and itching, protein kinase

stimulants, prophylactic/therapeutic agent for mitochondrial encephalomyopathy, prophylactic/therapeutic agent for arterial obstruction/stenosis, inhibitor of blood-brain barrier disruption, therapeutic agent for drug addiction, an apoptosis inhibitor, inhibitor of a lipid peroxide production, a radical scavenger, an amyloid β peptide aggregation inhibitor, and an amyloid β peptide injury inhibitor, Inhibitor of cholinesterase activity, serine/threonine kinase (Akt) activator, phosphatidylinositol (3,4,5) triphosphate kinase (PI3K) - serine/threonine kinase (Akt) cascade activator, promoter for increasing cyclic AMP concentration, or SAPK/JNK phosphorylation inhibitor. In addition, the anti-neurodegenerative disease agent of the present invention can also be used as a therapeutic or prophylactic agent for animals other than humans, including pets that have developed a neurodegenerative disease.

Example 4

<Liquid agent for injection>

A solution, the pH of which is adjusted to 7.2, prepared by dissolving 50 g of pyrogen-free hydrous crystalline α,α-trehalose ("TREHALOSE SG", commercialized by Hayashibara Co., Ltd., Okayama, Japan), 0.5 g of ascorbic acid, and 1.25 g of sodium hydrogen carbonate in 370 g of purified water for injection; and another solution prepared by dissolving 3 g of Tween 80 ("POLYSORBATE 80", commercialized by Nippon Oil & Fats Co., Ltd., Tokyo, Japan) and 120 mg of a compound selected from NK-6 and its analogs, represented by Chemical formulae 7 to 11 (NK-1946, NK-1945, NK-10745, and NK-1486) (all manufactured by Hayashibara Co., Ltd., Okayama, Japan) as an active ingredient in 177 g of purified water for injection; were mixed. The resulting mixture was sterilized by filtering and bubbled aseptic nitrogen gas to give a concentration of dissolved oxygen of about 0.1 ppm. One milliliter each of the resulting solution was dispensed into brown ampoules, and the ampoules were sealed under a nitrogen stream. The products are pyrogen-free and can be used as an anti-neurodegenerative disease agent for injection as in the case of Example 4 in the scope of application of the example.

Example 5

<Powder agent to be dissolved at use>

A solution, the pH of which is adjusted to 7.0, prepared by dissolving 30 g of purified maltose for injection (manufactured by Hayashibara Co., Ltd., Okayama, Japan), 0.5 g of ascorbic acid, and 1 g of sodium hydrogen carbonate in 370 g of purified water for injection; and another solution prepared by dissolving 200 mg of a compound selected from NK-5 and its analogs, represented by Chemical formulae 2 to 6 (NK-36, NK-37, KK-44, and NK-45) and NK-6 and its analogs, represented by Chemical formulae 7 to 11 (NK-1946, NK-1945, NK-10745, and NK-1486) (all manufactured by Hayashibara Co., Ltd., Okayama, Japan) in 100 g of purified water for injection; were mixed. After sterilizing the resulting mixture by filtering, 10 mL each of the resulting solution was dispensed into brown ampoules and freeze-dried by a conventional method, and the ampoules were sealed under a nitrogen stream. The products are pyrogen-free, and can be used for intravenous instillation, subcutaneous administration, intraperitoneal administration, or the like, after dissolving the products by adding 2 to 10 mL of purified water for injection or physiological saline at the time of use. The products are in a dosage form suitable for dye compounds that are inferior in stability in an aqueous solution, and it can be used as an anti-neurodegenerative disease agent for injection as in the case of Example 4 in the scope of application of the example.

Industrial Applicability

The anti-neurodegenerative disease agent of the present invention is useful for prevention, treatment and/or suppression of progression of diseases caused by neurodegeneration of nerve cells, and protection of nerve cells from factors causing neurodegeneration. It is also useful for improving various pathological conditions and neurological dysfunction associated with neurodegenerative diseases. Furthermore, the anti-neurodegenerative disease agent of the present invention has high solubility and improved brain permeability, so that it can be orally administered. It can be also expected that even a smaller dose of administration has the same or higher effect as the conventional agents, and it increases the degree of freedom when formulating into aqueous preparations, therefore, the present invention has a remarkable inventive step. The present invention with such an outstanding function and effect is a significant invention that will greatly contribute to this art.

## Claims

1. An anti-neurodegenerative disease agent comprising a compound represented by following General formula 4 as an active ingredient, and having an action of accelerating neurocyte growth of the central nervous system, an action of promoting neurite-outgrowth, and an action of scavenging reactive oxygen species;

General formula 4:

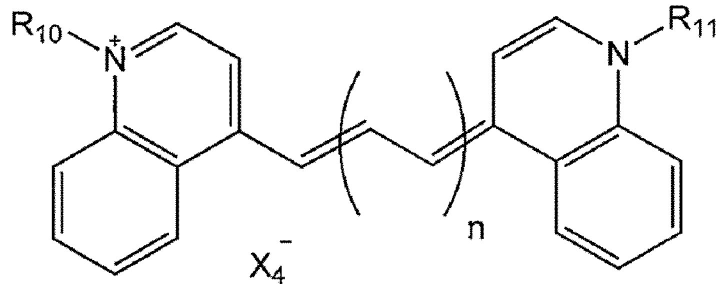

wherein General formula 4, R10 and R11 each independently represent the same or differing alkyl groups having a carbon number of 2 to 8; alkyl groups may be linear or branched; n represents an integer of 0 or 1; and $X_4^-$ represents an appropriate, pharmaceutically acceptable counter anion.

2. The anti-neurodegenerative disease agent of claim 1, wherein said anti-neurodegenerative disease agent is an orally administered agent.

3. The anti-neurodegenerative disease agent of claim 1 or 2, wherein a molecular weight of a cationic portion of the compound represented by General formula 4 is less than 500 Da.

4. The anti-neurodegenerative disease agent of any one of claims 1 to 3, wherein said neurodegenerative disease is a neurodegenerative disease selected from the group consisting of Alzheimer's disease, Parkinson's disease, dementia, spinocerebellar degeneration, cerebral infarction and ataxia.

5. The anti-neurodegenerative disease agent of any one of claims 1 to 4, wherein the counter anion of the compound represented by General formula 4 is any one of iodine ion, bromine ion or chlorine ion.

6. The anti-neurodegenerative disease agent of any one of claims 1 to 5, wherein the compound represented by General formula 4 is a compound represented by any one of following Chemical formulae 2 to 11:

Chemical formula 2:

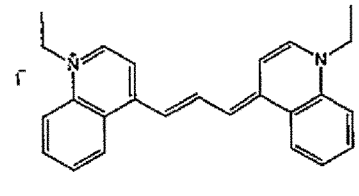

Chemical formula 3:

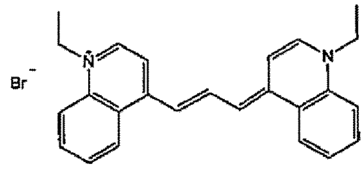

Chemical formula 4:

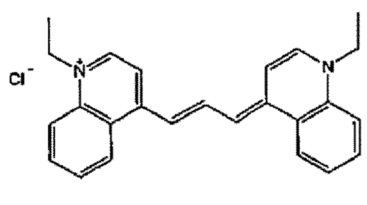

Chemical formula 5:

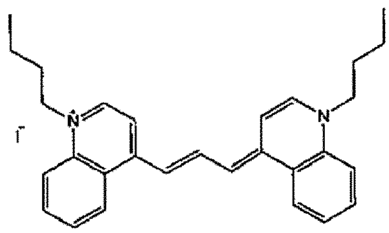

Chemical formula 6:

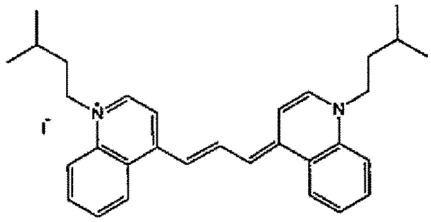

Chemical formula 7:

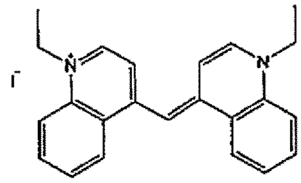

Chemical formula 8:

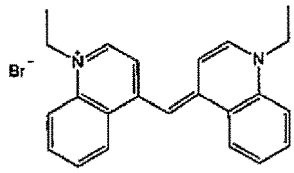

Chemical formula 9:

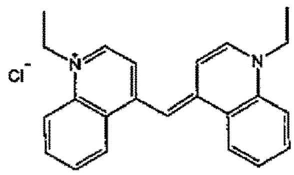

Chemical formula 10:

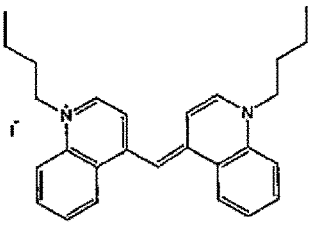

Chemical formula 11:

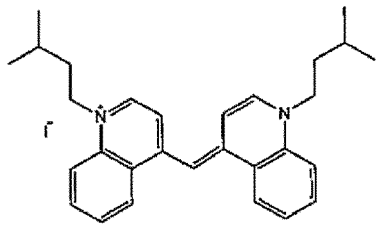

7. The anti-neurodegenerative disease agent of any one of claims 1 to 6, said anti-neurodegenerative agent are the compounds represented by General formula 4; wherein said anti-neurodegenerative agent comprises (a) and (b) as active ingredients:

(a) one or more compounds in which n is 1; and
(b) one or more compounds in which n is 0;

and wherein the molar ratio of (a) : (b) is in the range of 10:1 to 1:10.

**INTERNATIONAL SEARCH REPORT**

International application No. PCT/JP2019/037352

A. CLASSIFICATION OF SUBJECT MATTER
Int.Cl. A61K31/4709(2006.01)i, A61P9/10(2006.01)i, A61P25/16(2006.01)i, A61P25/28(2006.01)i, A61P39/06(2006.01)i, A61P43/00(2006.01)i

According to International Patent Classification (IPC) or to both national classification and IPC

B. FIELDS SEARCHED

Minimum documentation searched (classification system followed by classification symbols)
Int.Cl. A61K31/4709, A61P9/10, A61P25/16, A61P25/28, A61P39/06, A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| A | JP 2014-101358 A (MIE UNIVERSITY et al.) 05 June 2014, examples, compound 46 & US 2015/0182518 A1, examples, compound 46 & WO 2014/065439 A1 & EP 2849743 A1 | 1-7 |
| A | JP 2001-512448 A (OSSWALD, Hartmut) 21 August 2001, claims, examples & WO 1998/035678 A1, claims, examples & EP 949924 A1 & DE 19706161 A1 | 1-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 October 2019 (30.10.2019) | 12 November 2019 (12.11.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/037352

C (Continuation). DOCUMENTS CONSIDERED TO BE RELEVANT

| Category* | Citation of document, with indication, where appropriate, of the relevant passages | Relevant to claim No. |
|---|---|---|
| A | RUSS, H. et al., "Cyanine-related compounds: a novel class of potent inhibitors of extraneuronal noradrenaline transport", Naunyn-Schmiedeberg's Arch Pharmacol., 1993, vol. 348, issue 5, pp. 458-465, in particular, fig. 1, table 3 | 1-7 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 97030703 A
- JP 11228417 A
- JP 2006143708 A
- JP 2006321737 A
- JP 2002234841 A
- WO 2010087306 A


### Non-patent literature cited in the description


- Kankoso-Hyo. Table of Photosensitive Dyes. Kankoshikiso Kenkyu-Sho, 1969
- *CHEMICAL ABSTRACT Index Guide (N-Z),* 1994, 1531G-1536G
- Photosensitive dyes -their mysterious actions and various functions. Sangyo Tosho Co., Ltd, 1997, 24-30
- The Chemistry of Synthetic Dyes. Academic Press, 1952, vol. 2, 1143
- THE CHEMISTRY OF SYNTHETIC DYES. 1971, vol. 4, 212
- *Therapeutic Delivery,* 2015, vol. 6 (7), 961-971
- *Analytical Chemistry (BUNSEKI KAGAKU),* 2004, vol. 53 (9), 953-958
- Measurement of partition coefficients (1-octanol/water)-Flask shaking method. *Japanese Industrial Standards,* 2000, Z7260-107
- *Drug Metabolism and Disposition,* 2004, vol. 32 (1), 132-139
- *Journal of Pharmaceutical Science,* 2009, vol. 98 (1), 122-134
- *Pharmacia,* 2012, vol. 48 (8), 761-766
- TREHALOSE 100PH. Hayashibara Co., Ltd,